# EUROPEAN PATENT SPECIFICATION

(11) **EP 3 434 256 B1**
(45) Date of publication and mention of the grant of the patent: **12.11.2025**
(21) Application number: 17183285.0
(22) Date of filing: 26.07.2017
(51) Int. Cl.: A61K 8/9789, A61K 36/28, A61K 36/287, A61Q 19/08, A61Q 19/02

(54) **TOPICAL HERBAL COMPOSITIONS**
TOPISCHE PFLANZLISCHE ZUSAMMENSETZUNGEN
COMPOSITIONS À BASE DE PLANTES POUR USAGE TOPIQUE

(43) Date of publication of application: 30.01.2019
(73) Proprietor: Chemisches Laboratorium Dr. Kurt Richter GmbH, 12277 Berlin (DE)
(72) Inventor: JOHN, Sabrina, 12277 Berlin (DE); PRADE, Heiko, 12277 Berlin (DE); VAN DER HOEVEN, Harald, 12277 Berlin (DE)
(74) Representative: Vossius & Partner Patentanwälte Rechtsanwälte mbB

(56) References cited:
- EP-A1- 1 737 538
- EP-B1- 1 737 538
- CA-A1- 2 706 992
- JP-A- 2011 213 699
- US-A- 5 869 031
- US-A- 6 159 487
- US-A1- 2008 050 459
- US-A1- 2009 028 826

## Description

### FIELD OF THE INVENTION

The present invention pertains to topical formulations having an autophagy stimulating and/or heme oxygenase and activating/triggering effect on human skin for cosmetic and pharmaceutical use. The topical formulations of the invention also trigger production of VEGF-C, reduce melanin synthesis and boost energy and cell function.

Moreover, the present invention also provides a new process for producing a herbal composition comprising a mixture of components of *Bellis perennis L.* and optionally *Hieracium pilosella* by aqueous extraction and the herbal composition comprising a mixture of components produced by the process of the present invention.

The pharmaceutical formulations according to the present invention may be used for the treatment or prevention of dark circles under the eye and/or puffy eyes.

The herbal composition according to the present invention may also be used in a cosmetic formulation for the cosmetic treatment or prevention of dark circles under the eye and/or puffy eyes

### BACKGROUND OF THE INVENTION

Dark circles under the eyes are a common phenomenon. Dark circles make people look tired and older and can have significant impact on quality of life. Men and women are equally affected and, although aging plays a causative role, dark circles are seen at all ages.

The pathophysiology of dark circles is multifactorial and different physiological and pathophysiological processes as well as environmental stimuli may be implicated. In reducing the appearance of dark circles it is therefore essential to find common causes for the different processes which lead to the formation of dark circles. Aging related factors are important and need to be addressed, but the processes behind the daily fluctuations of dark circles are at least as important.

As set out below, various factors may contribute to the optical appearance of dark circles, which may include factors such as increased skin translucency, increased skin laxity and pigmentation, decreased lymphatic drainage and hemocongestion.

The skin under and around the eyes is particularly thin. The aging processes, both biological and accelerated by sunlight, play an important role in further thinning of the skin, where collagen and other components of the connective tissue are broken down, leading to the decline and destruction of the subcutis. Due to the decreasing thickness of the skin, the skin becomes increasingly translucent. This is a particular problem for the skin under the eyes, as here, we find a vast network of capillary blood vessels, lymph vessels and muscles just below the surface of the skin. As a consequence, thinning of skin leads to a darkened appearance, a factor playing an eminent role in the appearance of dark circles.

Due to aging, skin becomes lax and gravity will pull the lower eyelids down, leading to a shadowing effect, which is thus a further contributor to the appearance of dark circles. Additionally, due to the increase in skin laxity, skin becomes stretched and therefore even thinner and more translucent.

Damage, caused by UV light, can lead to hyperpigmentation. Especially people with a higher skin phototype often show a phenomenon called postinflammatory hyperpigmentation. This can play a prominent role in the appearance of dark circles. The hyperpigmentary factor in the appearance of dark circles is not just an epidermal phenomenon. Dermal melanin deposition, where melanin-containing melanosomes have been engulfed in macrophages, resulting in the formation of so-called melanophages, is described to play an important role in the appearance of dark circles.

The most important function of the lymph vessels is to maintain a balance in fluid, macromolecules and oncotic pressure in the interstitial areas (extracellular space) of our body. They drain excess tissue fluid back to the blood circulation. Macromolecules and cells can directly enter the lymphatic vessels.

The lymphatic vessels become reduced in number and become increasingly hyperpermeable during the aging process as well as consequential to inflammatory processes. This results in edema of the lower eyelid, an accumulation of fluid. This fluid often takes on a purplish color and can significantly influence the color of skin under the eyes.

As mentioned above, another important factor in the etiology of dark circles, specifically related to aging, is dermal melanin deposition in melanophages. The cutaneous lymph vessels are extremely important in trafficking macrophages out of the skin. Melanophages are macrophages containing melanin, another illustration of the importance of the cutaneous lymphatic system in the appearance of dark circles.

Blood flow in the under eye-area is sluggish and slow. Hemocongestion, where blood flow is reduced to zero, is an often-occurring phenomenon in the skin under the eyes. Oxygenated hemoglobin has a reddish color and produces a pinkish tint in the skin. In contrast, deoxygenated hemoglobin has a purplish color and produced a tint which is more bluish. Hemocongestion is associated with a large presence of deoxygenated hemoglobin and strongly contributes to the appearance of dark circles.

Consequential to hemocongestion, but also to the inflammatory and aging processes, vascular permeability is increased. In the interstitial area in the dermis, the leaked red blood cells burst, releasing hemoglobin. The hemoglobin rapidly releases its heme group. The color of heme is very dark and can contribute to the appearance of dark circles significantly. Additionally, heme is a molecule which can induce a multitude of deleterious reactions in the dermis, many of them relevant for the formation and maintenance of dark circles.

Heme can cause cell damage, especially relevant for epithelial cells of both the cutaneous blood microvasculature and the lymph vessels. Heme induces oxidative stress, the breakdown of the extracellular matrix in the dermis (collagens, elastin, etc.) and stands at the beginning of inflammatory processes, supporting the pathophysiology of the formation of dark circles.

The inflammatory processes initiated by heme result in a further increase in permeability of both blood and lymph vessels. Heme is therefore a major contributing factor in the etiology of dark circles, both of the aging-related type and the acute type, the type which can change in appearance day to day.

Puffiness or bagginess under the eyes with associated discolorations also has many etiologic factors including an abnormal increase in leakage from capillaries beneath the surface of the skin. Fluid accumulating beneath the skin in the region under the eyes results in edema which manifests as baggy eyes often relatively darker in color. Puffy or baggy eyes are perceived as being cosmetically unacceptable or may be present to such an extent that therapeutic intervention is necessary. This may particularly be the case in connection with allergies, hay fever and the like.

The exact reasons for such increased capillary permeability is not always known, but several factors such as stress, allergic reactions, kidney malfunctions, high blood pressure, water retention, excessive consumption of caffeine and lack of sleep have been identified as being associated with the problem. Intrinsic aging and photodamage can also lead to similar changes.

It is the eyes that attract focal attention, and therefore it is desirable to avoid or reduce discoloration and puffy or baggy eyes in order to retain a cosmetically acceptable appearance.

People often resort to cosmetic surgery to remove bags beneath the eyes and restore the smoothness of the lower eyelids. The problems with cosmetic surgery include its great expense and risks of anesthesia and infection that accompany every surgical procedure.

Cosmetic products for application to the skin beneath the eye contain ingredients, such as plant extracts, aimed solely at making the lower eyelids feel comfortable and soothed. This merely amounts to relieving surface irritations, and does not address the cosmetic problems of the skin in order to restore elasticity and firmness to puffy skin beneath the eye.

Accordingly, there is a need for a product that is effective in (a) retarding formation of bags beneath the eyes, (b) partially or fully restoring a smooth skin contour to puffy skin beneath the eye, and (c) minimize dark circles under the eyes, thereby leading to a youthful appearance of the area around the eyes.

*Bellis perennis L.,* commonly known as English daisy or lawn daisy, belongs to the family of the Asteraceae.

It is widely used in homeopathy for treating different symptom complexes such as arthrosis, loss of appetite and sleeping disorders. Moreover, *Bellis perennis L.* is also traditionally used for the treatment of dermatological problems such as acne, eczema, badly healing wounds and deeper traumata of the tissue (see, e.g., H.A. Hoppe, Drogenkunde, Vol. 1, Angiospermen, 8. Edt., 1975; Dr. F. Losch, Kräuterbuch; G. Leibold, Moderne Naturheilpraxis, Bassermann Verlag, 1993; M. Lange-Ernst, S. Ernst, Lexikon der Heilpflanzen, Honos Verlag; and W.D. Storl, Heilkräuter und Zauberpflanzen, AT Verlag, 2. Edt., 2000).

More recently *Bellis perennis L.* has been subject of pharmacological investigations, and particular ingredients, such as triterpene glycosides, have been identified to exhibit a broad pharmacological activity profile such as antifungal and antimicrobial, anticancerogenic and also post ischemic neuroprotective effects (see, e.g., DE 42 06 233; US 6,444,233; G. Bader et al., Pharmazie 1990 Jul; 45(8); P. Avato et al.; Planta Med 1997 Dec; 63(6); and C. Desevedavy et al.; Journal Nat Prod 1989 Jan-Feb; 52(1)).

*Hieracium pilosella (syn. Pilosella officinarum),* also known as mouse-ear hawkweed, is a yellow-flowered species of flowering plant in the daisy family Asteraceae, native to Europe and northern Asia. It produces single, lemon-colored inflorescences. It is an allelopathic plant. Like most hawkweed species, it is highly variable and is a member of a species complex of several dozens of subspecies and hundreds of varieties and forms.

*Hieracium pilosella* contains umbelliferone, a compound similar to coumarin and a known antibiotic against brucellosis, as well as a frequent active compound in sunscreen lotions. The plant is also a potent diuretic and has been suggested/used as a further (adjuvating) component in formulations containing i. a. *Ruscus aculeatus,* caffeine tocopherol nicotinate, *Aesculus hippocastanum* or capsaicin, for the treatment of cellulite (WO 2004/110396, FR 2 729 856, WO 2012/052685).

EP 790 054 discloses compositions for topical treatment of skin problems associated with abnormal pigmentation. More particularly, this document focuses on the use of live yeast cell derivative in combination with a suitable vehicle, for treating discolorations and bagginess in facial skin below the eyes.

US 5,204,105 pertains to agents for reducing puffiness of the skin under the eyes and for reducing sensations of irritation and inflammation of the skin under the eyes selected from the group consisting of plant extracts and yeast extracts and combinations thereof. The plant extracts can comprise *Ruscus aculeatus* (butcher broom), hydrocotyl, *Aesculus hippocastanum* (horse chestnut), *Calendula officinalis* (calendula), *Hamamelis virginiana L.* (hamamelis, witch-hazel), *Equisetum arvense L.* (equisetum, horsetail), Euphrasia (eyebright), *Prunus persica* (peach), *Alchemilla vulgaris* (lady's mantle), *Hedera helix (ivy), Matricaria chamomilla* (German chamomile), and *Symphytum* (comfrey).

JP 2011 213699 A discloses external skin preparations comprising an extract of *Bellis perennis* for stimulation of collagen production.

CA 2 706 992 A1 relates to topical cosmetic formulations for skin lightening comprising an extract of *Bellis perennis, a Phyllanthus* extract and a *Glycyrrhiza* extract.

A cosmetic preparation comprising a *Phyllanthus* extract for treating of fine lines and wrinkles and/or skin brightening is disclosed in US 2008/050459 A1.

US 6 159 487 A discloses a cosmetic product, especially for placement over the eyes for reducing puffiness, dark circles, redness and combinations thereof.

### SUMMARY OF THE INVENTION

There is thus a need for compositions effectively reducing dark circles and also puffiness which focus on physiological events standing at the crossroads of the biological processes inducing the formation of dark circles and puffiness, such as heme.

The present invention provides pharmaceutical and cosmetic formulations particularly directed to satisfying this need. The formulations according to the present invention reduce puffy skin beneath the eye, smooth the contour of the skin, and reduce dark circles under the eye.

The herbal compositions and topical formulations according to the present invention comprising components derived from *Bellis perennis and Hieracium pilosella* address the most important aspects in the formation and the maintenance of dark circles.

The herbal composition according to the present invention induces the production of heme oxygenase and VEGF-C, activates autophagy and increases cellular functionality and energy. It also reduces melanin synthesis. The appearance of dark circles, their color and surface area are thus reduced, leading to a youthful appearance of the area around the eyes.

### BRIEF DESCRIPTION OF THE FIGURES

- **Figure 1:**: Shows the promotion of the expression of HO-1 by keratinocytes by the herbal composition.
- **Figure 2:**: Shows the promotion of the expression of HO-1, despite prolonged exposure to heme by the herbal composition.
- **Figure 3:**: Shows the increase of both metabolic activity and energy production in keratinocytes by the herbal composition.
- **Figure 4:**: Shows the promotion of autophagy by the herbal composition.
- **Figure 5;**: Shows the reduction of melanin production in epidermal skin models containing melanocytes, stressed by heme and UV radiation by the herbal composition.
- **Figure 6:**: Shows the increases of VEGF-C production by lymphatic endothelial cells by the herbal composition.
- **Figure 7:**: Shows the increases of VEGF-C production by lymphatic endothelial cells by the herbal composition under influence of TGF-β.
- **Figure 8:**: Shows that the herbal composition performs better than placebo in reducing the surface area of dark circles (*in vivo*).
- **Figure 9:**: Shows that the herbal composition leads to a stronger reduction of the surface area of dark circles than placebo. Results obtained on 12 volunteers, after 56 days, twice daily application (*in vivo*).
- **Figure 10:**: Shows that the herbal composition leads to a stronger reduction of the color of dark circles than placebo. Results obtained on 12 volunteers, after 56 days, twice daily application (*in vivo*).

### DETAILED DESCRIPTION OF THE INVENTION

As already mentioned above, the present invention is based on the unexpected finding that components derived from *Bellis perennis and Hieracium pilosella* synergistically induce the production of heme oxygenase and VEGF-C, activate autophagy and increase cellular functionality and energy and also reduce melanin synthesis and as such are ideally suited for the treatment and prevention of dark circles under the eyes as well as puffiness.

Thus, the present invention is as defined in appended claims 1 to 25.

### HERBAL COMPOSITION ACCORDING TO THE INVENTION AND ITS PREPARATION

Herbal composition comprising mixture of components obtained from the plants according to the present invention.

The components are obtained from fresh or dried plant material of *Bellis perennis* and *Hieracium pilosella.*

The herbal composition comprises a mixture of components obtained from *Bellis perennis* and *Hieracium pilosella.*

### Bellis perennis

The mixture of components obtained from *Bellis perennis* may be prepared from fresh or dried plant material such as flower heads, whole plant or aerial parts of the plant. The plant material may be milled, comminuted or used unground/unbroken.

The use of dried aerial parts of the plant or dried flower heads of *Bellis perennis* is preferred. Most preferred is the use of the dried and milled flower heads of *Bellis perennis*

### Hieracium pilosella

The mixture of components obtained from *Hieracium pilosella* may be prepared from fresh or dried plant material such as flower heads, whole plant or aerial parts of the plant. The plant material may be milled, comminuted or used unground/unbroken.

The use of dried aerial parts of the plant or dried whole plant of *Hieracium pilosella* is preferred. Most preferred is the use of the dried and milled aerial parts of the plant of *Hieracium pilosella.*

The mixture of components obtained from *Bellis perennis* and *Hieracium pilosella,* respectively, may be prepared by any appropriate method known to the person of skills in the art, whereby the components are obtained by extraction with a citrate buffer pH 5.0.

### Solvent extraction

According to the present invention, any extract of *Bellis perennis L.* and *Hieracium pilosella* obtained by decoction, digestion, percolation, soxlethtation, maceration or any other appropriate extraction method with a citrate buffer pH 5.0 known to the person of skills in the art may be used.

### Extraction media

As stated above, the extraction media according to the present invention is a citrate buffer pH 5.0.

In a more preferred embodiment, a sodium-citrate buffer according to Sörensen (buffer containing 0.1 M disodium citrate and 0.1 N HCl, having a pH 5.0) is used. In a further preferred embodiment, a phosphate buffer according to Sörensen (0.06 M potassium phosphate and 0.06 M disodium phosphate pH 5) is used.

In an even more preferred embodiment, a sodium citrate buffer according to Sörensen with 0.1 M citric acid monohydrate (C₆H₈O₇xH₂O) and 0.1 M trisodium citrate dihydrate solution having a pH of 5.0 is used.

### Ratio drug and extraction medium

The proportion between drug and extraction medium (solvent) ranges from 1:100 (1% w/w) to 20:100 (20% w/w), 1:100 (1% w/w) to 50:100 (50% w/w) preferred from 3:100 to 10:100 or 3:100 to 30:100, more preferred from 5:100 to 10:100 or 5:100 to 7:100.

It is particularly preferred that the proportion between drug and extraction medium is between 5:100 and 10:100, preferably 5:100 to 7:100.

### Ratio of components in the herbal composition

According to the present invention, the mixture of components obtained from *Bellis perennis* and *Hieracium pilosella* are present in the herbal composition in a ratio *(Bellis:Hieracium)* in the range of 1:1 to 1:9, preferably 1:1 to 3:1 or 1:1 to 5:2 , more preferably 1:1 to 2:1 or 1:1 to 3:1, most preferred with respect to activity and/or stability are 1:1 and 3:1.

### PH value of the final herbal composition

The herbal composition of the present invention has a pH value of between about pH 3 and pH 7, preferably in the range of pH 4.0 and pH 6.5.

As stated above, the herbal composition according to the present invention comprising a mixture of components obtained from *Bellis perennis* and *Hieracium pilosella* which may be prepared by separate preparation from the respective plant (i.e. obtaining the components from each plant separately and combining the obtained mixtures of components afterwards) or by obtaining the mixture of components jointly from a mixture of the starting plants.

A further object of the present invention is to provide a process for producing an aqueous extract of *Bellis perennis L.* and an extract obtained by that process.

The mixture of components obtained from *Bellis perennis* may i. a. be obtained in accordance with the process and methods disclosed in EP 1 737 538.

According to the invention, the extraction time and temperature may vary depending on the extraction media, particularly an extraction time between 1 h and 10 days. More particularly, a time between 1 h and 24 h, 2 h and 8 h, 1 h and 4 h or 2 h and 4 h is preferred.

The temperature may range between 20°C and 100°C, preferably between 25°C and 85°C or between 25°C and 60°C.

In a particularly preferred embodiment of the present invention, *Bellis perennis* and *Hieracium pilosella* are separately extracted with citrate buffer pH 5.0, such as the Sörensen citrate buffer described above.

### Exemplary process

It is particularly preferred that *Bellis perennis* (in particular dried flower heads of *Bellis perennis)* are extracted with citrate buffer pH 5.0 at a temperature of 60°C for 4 to 8 hours, whereby the proportion of drug (i.e. plant):extraction medium is 7:100 (7%) to 10:100 (10%). After extraction the plant material is separated from the liquid phase, e.g., by a decanter CA 22 (Westfalia Seperator AG).

It is, moreover, particularly preferred that *Hieracium pilosella* (in particular the dried whole aerial part of the plant) are extracted with citrate buffer pH 5.0 at a temperature of 25°C to 60°C for 2 to 4 hours., whereby the proportion of drug:extraction medium is 5:100 (5%) to 7:100 (7%). The crude extract is then fractionated by ultrafiltration (MW cut-off: 100 kDa) to obtain the < 100 kDa fraction.

The <100 kDa fraction is used for preparing the herbal composition by combining it with the mixture of components obtained from *Bellis perennis* at the ratio in accordance with the present invention.

### Preservation of the herbal composition

The herbal composition according to the present invention may be expediently preserved by the addition of preservatives commonly used in the art such as sodium dehydroacetate, 2-hydroxybenzoic acid, 1-phenyl-1-propanol, Dermosoft^{®} 700B (mixture of levulinic acid; sodium levulinate; glycerin and water), phenethyl alcohol or sodium benzoate at concentrations commonly known in the art such as 0.02% (w/w), 0.2% 0.3% (w/w), 0.5% (w/w) 0.85% (w/w) and 0.5% (w/w), respectively.

The use of sodium dehydroacetate and/or sodium benzoate is particularly preferred, whereby a concentration in the range of 0.2% and 0.5% (w/w) is preferred.

### Stabilisation of the herbal composition by fermentation

In a further embodiment of the present invention, at least one of the mixtures of components obtained from *Bellis perennis* and *Hieracium pilosella* or the herbal composition may optionally be subjected to fermentation.

The such obtained herbal composition may exhibit an enhanced stability and activity, such as enhanced endothelin-1 activity.

It is preferable that said fermentation is *Lactobacillus, Bifidobacter* or *Lactococcus* fermentation.

To this effect the following subspecies are preferred:
*Bifidobacter sp. longum, Lactococcus sp. Lactis and Lactobacillus sp. Helveticus.*

For the fermentation according to the present invention the bacteria of choice are first cultivated in a nutrition medium in order to obtain an inoculum which is then added to a liquid mixture of components obtained from from *Bellis perennis* and/or *Hieracium pilosella* or the herbal composition.

The fermentation is carried out under anaerob conditions for about 24 to 48 hours at a temperature of about 32 to 37°C.

Fermentation is stopped by inactivation of the bacteria by pasteurization, such as heat inactivation of the bacteria at 85°C for about 45 min.

The following process serves for illustrative purposes only and should not be construed to be limiting.

### Exemplary fermentation process

*Lactococcus sp.* were cultivated in nutrition medium having the following composition:

| | (g/l) |
|---|---|
| Wheat Pepton | 7.0 |
| Yeast extract | 3 |
| K₂HPO₄ | 2 |
| NaCl p.A. | 1 |
| Glucose Monohydrate | 5.5 |
| Fructose | 5 |
| Water | 976.5 |
| + trace elements solution | 1 |
| total | 1000.00 |

Trace elements solution:

| | g/20 ml |
|---|---|
| MgSO₄ x7H₂O | 1.333 |
| MnCl₂x4H₂0 | 0.293 |
| Water | 18.37 |

An extract of *Bellis perennis* and *Hieracium pilosella* was prepared by (e.g.) 10% dried drug in water for 1 hour at 95°C or in accordance with the processes above by combined or separate extraction, preferably by combined extraction.

The biomass was separated and the liquid phase served as the nutrition medium for the bacteria. In a further embodiment, biomass and liquid phase were not separated after extraction and the obtained suspension served as the nutrition medium for the bacteria.

Fermentation under anaerobic conditions was carried out for 48 hours at 36°C using *a Lactobacillus* inoculum. After this time the fermentation was finished by heat inactivation of the bacteria (45 minutes, at 85°C).

### CHARACTERIZATION OF THE HERBAL COMPOSITION

### Characterization of herbal composition by LC-HRMS

Qualitative characterization of the herbal composition of the invention by its components was carried out by liquid chromatography - high resolution mass spectrometry (LC-HRMS).

In short, the herbal composition of the invention was diluted in water and analyzed by a reversed-phase capillary liquid chromatography system (Dionex Ultimate 3,000 NCS-3500RS Nano, Thermo Scientific) connected to an Orbitrap Fusion mass spectrometer (Thermo Scientific).

In a preferred embodiment, the herbal composition according to the present invention (such as F111) comprises the following components as shown in Table 1, below:

**Table 1: Preferred qualitative composition of the herbal composition of the invention**

| **Compound/components** | **mass [M+H]⁺** | **Herbal composition according to the present invention** |
|---|---|---|
| Chlorogenic acid | 353.0867 | x |
| | | |

| Anthocyanidines | | |
|---|---|---|
| Cyanidin 3-malonylglucoside | 535.1082 | x |
| Cyanidin 3-glucuronylglucoside | | |
| | | |

| Flavonoides | | |
|---|---|---|
| Apigeninhexoside | 433.1129 | x |
| Apigeninglucuronide | 447.0922 | x |
| Apigeninmethylglucuronide | 461.1078 | x |
| Kaempferolhexoside | 449.1078 | x |
| Quercetinhexoside | 465.1027 | x |
| Isorhamnetinhexoside | 479.1184 | x |
| Apigeninglucoside | 489.1027 | x |
| Isorhamnetinglucuronide | 493.0977 | x |
| Isorhamnetinglucoside | 565.1188 | x |
| Isorhamnetin | | x |
| | | |

| Saponines | | |
|---|---|---|
| Perennisoside III, IV, V, VI | 1163.5844 | x |
| Perennisoside XII | 1121.5738 | x |
| Perennisoside XIII | 1207.6106 | x |
| Perennisoside XIV | 1283.6266 | x |
| Perennisoside XV | 1369.6634 | x |
| Perennisoside XVIII & XIX | 1105.5789 | x |
| | | |
| Desacyl-perennisoside I, II & III | 959.5210 | x |
| Desacyl-perennisoside VIII & IX | 1105.5789 | x |
| Desacyl-perennisoside X & XI | 1283.6266 | x |
| | | |
| | | |

The saponine compounds shown in Table 2, below, are optional components of the herbal compositions according to the present invention:

**Table 2. Optional components of the herbal composition of the invention**

| **Compound/components** | **mass [M+H]⁺** |
|---|---|
| Saponines | |
| Bellisisosides C & E | 1307.6630 |
| Bellisisoside D | 1349.6736 |
| Perennisosides I & II | 1043.5421 |
| Perennisoside VII | 1205.5950 |
| Perennisosides VIII & IX | 1189.6000 |
| Perennisosides X & XI | 1367.6478 |
| Perennisosides XVI | 1325.6372 |
| Perennisaponine A | 1159.5895 |
| Perennisaponines G; H | 1305.6474 |

### DRY CONTENT OF THE HERBAL COMPOSITION OF THE INVENTION

The herbal composition of the present invention is characterized by a dry content (incl. buffering agent and preservative) of about 3.5% to 5.5%, preferably about 4%. The dry water content was determined by the modified official method for the determination of water content according to the Official Collection, § 35 LMBG (German Law on Food and Consumer Goods) L 02.06-2 (EC).

The separate mixture of components obtained from *Bellis perennis* (i.e. prior to combination with the components of *Hieracium pilosella*) is characterized by a dry content (incl. buffering agent and preservatives) of about 4.3 to 5.3%, preferably of about 4.7%.

The separate mixture of components obtained from *Hieracium pilosella* (i.e. prior to combination with the components of *Bellis perennis*) is characterized by a dry content (incl. buffering agent and preservative) of about 3.3 to 4.3%, preferably of about 3.7%.

### PARTICULARLY PREFERRED EMBODIMENT

A preferred embodiment of the herbal composition according to the present invention is referred to in the present application as "F111" (INCI Name: Hieracium Pilosella (Hawkweed) Extract, Bellis Perennis (Daisy) Flower Extract).

This preferred embodiment is prepared by separate extraction with citrate buffer according to Sörensen (0.1 M citric acid monohydrate and 0.1 M trisodium citrate dehydrate) having a pH of 5.0 using the extraction methods described above.

This herbal composition exhibits a pH range of 4.0 - 6.5, and may preferably be preserved with preserving agents such a sodium dehydroacetate and/or sodium benzoate at concentrations commonly used in the art. It may be incorporated in a pharmaceutical/cosmetic composition/formulation preferably at a concentration of 3 - 5% (w/w) of the total weight of the total formulation.

### INDICATIONS

### Therapeutic application

The pharmaceutical formulation according to the present invention may be used in a therapeutic method of treating or preventing dark circles under the eyes (periorbital dark circles, periorbital hyperpigmentation, intraorbital venous stasis) and/or puffy eyes/puffiness (periorbital puffiness and/or periorbital edema).

The dark circles under the eyes and or puffiness may be caused by any one of heredity, allergies, sleep deprivation/fatigue, oversleeping, eczema (atopic dermatitis), contact dermatitis, hay fever/allergic rhinitis, stress, thinning skin and/or loss of fat and collagen with age (ageing), iron deficiency, minor trauma, crying, life style choices, fluid retention, skin pigmentation abnormalities (especially black and Asian people), excessive exposure to the sun, rubbing and scratching of eyes and medication.

### Cosmetic application

The cosmetic formulation according to the present invention may be used in a cosmetic method for the treatment or prevention of dark circles under the eyes and/or puffy eyes.

The dark circles under the eyes and or puffiness may be caused by any one of heredity, allergies, sleep deprivation/fatigue, oversleeping, eczema (atopic dermatitis), contact dermatitis, hay fever /allergic rhinitis, stress, thinning skin and/or loss of fat and collagen with age (ageing), iron deficiency, minor trauma, crying, life style choices, fluid retention, skin pigmentation abnormalities (especially black and Asian people), excessive exposure to the sun, rubbing and scratching of eyes and medication.

Preferred indications for the cosmetic formulation are dark circles under the eyes and puffiness due to thinning skin and/or loss of fat and collagen with age (ageing), life style choices, fluid retention, skin pigmentation abnormalities (especially black and Asian people), influence of circadian rhythm by life style and life conditions.

Other preferred cosmetic indications are dark circles under the eyes and puffiness due to sleep deprivation/fatigue, oversleeping, excessive exposure to the sun.

### TESTING OF THE COMPOSITION COMPRISING A MIXTURE OF COMPONENTS OF THE PLANTS ACCORDING TO THE PRESENT INVENTION (HERBAL COMPOSITION ACCORDING TO THE PRESENT INVENTION)

### Efficacy studies - in vitro assays

The following studies have been carried out with a herbal composition according to the present invention, **hereinafter called "F111",** without preservatives.

### "F111" may be characterized as follows:

| *INCI Name* | *CAS No.* | *EINECS No.* |
|---|---|---|
| Hieracium Pilosella (Hawkweed) Extract (EU Name: Hieracium Pilosella Extract) | 84012-22-6 | 281-668-2 |
| Bellis Perennis (Daisy) Flower Extract (EU Name: Bellis Perennis Extract) | 84776-11-4 | 283-935-9 |

| *Analytical Data* | | |
|---|---|---|
| Refractive index nD20 | | 1.3389 |
| Density 20°C | | 1.0191 g/ml |
| pH value | | 4.8 |
| Polyphenols | | 1850 mg/ml |
| Dry residue (with buffer, without preservatives) (2 h, 102°C) | | 3.6% |
| Color value (Gardner) | | 6.8 |

### Examples:

If not described otherwise, the data of the cell based assays have been normalized with the cell count obtained by DAPI staining.

### 1. Heme Oxygenase (HO-1)

Heme is broken down by Heme Oxygenase (HO-1). HO-1 is an enzyme strongly **involved in the skin's defense mechanisms against oxidative** stress. Heme is broken down by HO-1 to obtain biliverdin and biliverdin can be further broken down by biliverdin reductase to bilirubin. Heme is dark colored, biliverdin is rather greenish and bilirubin is yellowish.

HO-1 is inducible and produced by, for instance, by keratinocytes. Inducing HO-1 in keratinocytes is therefore an interesting approach towards accelerating the breakdown of heme. On top of the above-mentioned effects on skin color, the breakdown of heme is of great importance in stopping the progression of the deleterious processes induced by heme, as described above.

### Method

Human Keratinocytes (HaCaT) were treated with the test compounds (PBS and F111, respectively) for 48 hours. Determination of HO-1 was performed by ELISA (R&D Systems, Inc.; KCB3776). Relative fluorescence units were determined at 540 nm (ex.)/600 nm (em.). Non-treated cells are set at 100%.

### Results

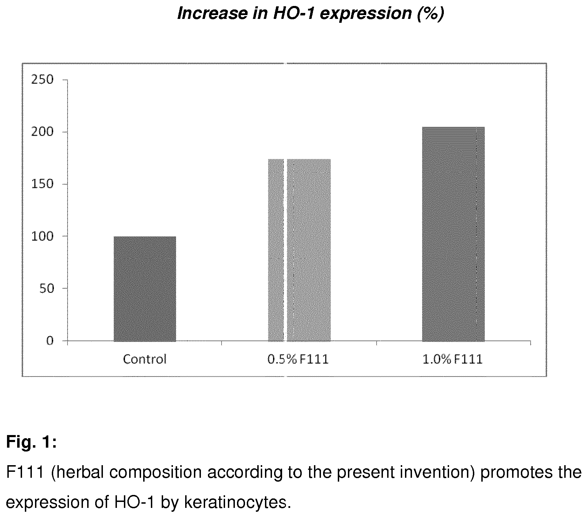

At different concentrations, F111 strongly promotes the production of HO-1 by immortalized keratinocytes (HaCaT) (**Fig. 1**). This indicates that F111 promotes heme degradation and supports one of the most important processes which act against the formation of dark circles.

### 2. Heme and HO-1 expression

Heme is pro-oxidative and pro-inflammatory. The presence of heme, therefore, can have a negative impact on skin cell functionality. As keratinocytes have the ability to produce HO-1, and can be exposed to heme, it is therefore important to verify that the effect of heme on HO-1 production by keratinocytes is not influenced negatively.

### Method

Human Keratinocytes (HaCaT) were pretreated with F111 for 24 hours. Heme (5 µM) was added for different periods of time. The determination of HO-1 was performed by ELISA (R&D Systems, Inc.; KCB3776). Relative fluorescence units were determined at 540 nm (ex.)/600 nm (em.). Non-treated cells are set at 100%.

### Results

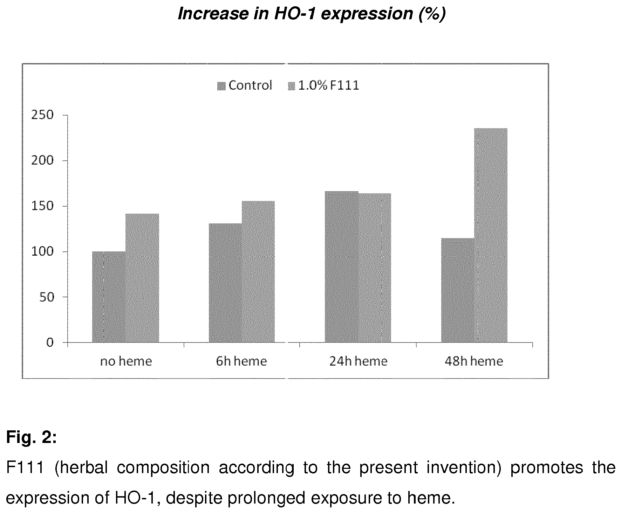

Heme treatment of immortalized keratinocytes (HaCaT) cells, at first, leads to an increase of production of HO-1, but prolonged exposure (48 h) to heme leads to a clear reduction of production of HO-1 (**Fig. 2**). As heme is proinflammatory and pro-oxidative, this is likely a result of the prolonged stress the cells are exposed to. F111 allows the cells to deal with the presence of heme, leading to an increased production of HO-1 after prolonged exposure to heme. This is contrary to untreated control cells. This experiment mimics the *in vivo* situation in dark circles where prolonged heme exposure can have deleterious effect on the skin cells and F111 shows here that it can compensate effectively for these effects.

### Heme oxygenase 1 (HO-1) assay for demonstration of synergistic effect

The inventors of the present invention conducted heme oxygenase 1 (HO-1) assays to demonstrate the synergistic effect provided by the combined components obtained from *Bellis perennis* and *Hieracium pilosella* (such as F111) as compared to the effects provided by the separate extracts of *Bellis perennis* and *Hieracium pilosella.*

The separate extracts of *Bellis perennis* and *Hieracium pilosella* were prepared with Sörensen citrate buffer pH 5.0 in accordance with the processes described above for the composition in accordance with the present invention, albeit the resulting liquid phases were not combined.

### Method

Human Keratinocytes (HaCaT) were pretreated with *Hieracium pilosella* extract or *Bellis perennis* extract or with F111 for 24 hours. Heme (5 µM) was added for different periods of time. The determination of HO-1 was performed by DuoSet^{®}IC total Heme Oxygenase 1 ELISA (R&D Systems, Inc.; DYC3776-2). Absorbance was determined at 450 nm (ex.)/570 nm (em.).

Non-treated cells were set at 100%.

### Results

### Synergystic effect promotes the expression of HO-1 on human keratinocytes after heme exposure (see Table 3, below)

The basal expression of HO-1 in keratinocytes is not inducible by treatment with *Hieracium pilosella* in lower concentrations.

A pronounced expression of HO-1 can be observed after application of an extract of *Bellis perennis* at all concentrations.

Treatment with F111 induced an even higher expression of HO-1, indicating a synergistic effect.

This effect is even more pronounced in the presence of damaging heme, in particular at 48 hours.

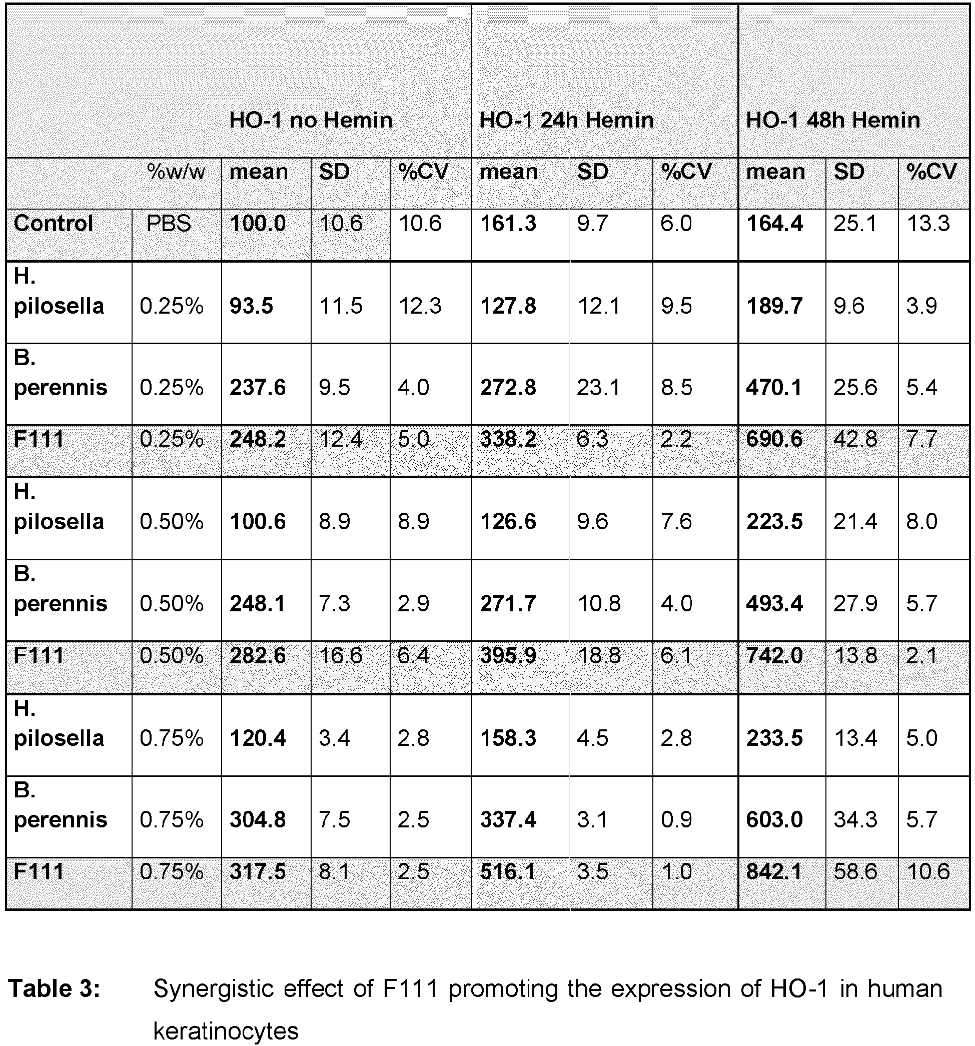

### 3. Cellular energy and metabolic turnover

Functionality of the skin cells, keratinocytes from the epidermis in particular, suffers with age and under inflammatory circumstances. Keratinocytes play an essential role in cell signaling. With that they stand at the basis of cell signaling towards the dermal compartment, the area in the skin where we find the extracellular matrix (collagens etc.) and blood and lymph vessels. Loss in keratinocyte functionality can have a negative impact on the structures in the dermal compartment and should therefore be addressed.

### Method

Human Keratinocytes (HaCaT) were pretreated with F111 for 24 hours. Determination of ATP was performed by Luminescent Adenosine Triphosphate Detection Assay (Packard Instrument Company, Inc.; 6016541). The luminescence was determined in a luminescence reader (Spectramax Paradigm, Molecular Devices, LLC.). The metabolic activity was measured by tetrazolium salt (MTT) assay. The absorbance was determined in a microplate reader (Spectramax Paradigm, Molecular Devices, LLC.) at 570 nm. Non-treated cells served as controls and were set to 100%.

### Results

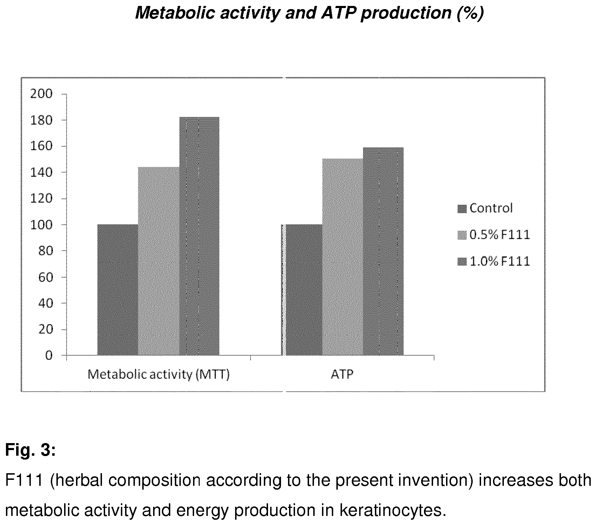

F111 at both 0.5% and 1.0% leads to an increase of metabolic activity and ATP (energy) production by immortalized keratinocytes (HaCaT) (Fig. 3). F111, therefore, shows to have a positive influence on these cells, increasing their functionality and health. At least part of the results obtained here can be attributed to the influence of F111 on autophagy, described below. One of the key features in dark circles is cellular stress, which can have a significant impact on cell functionality. The results from this experiment show that F111 supports the skin cells in compensating for this stress.

### 4. Autophagy

Autophagy is a process with which cells can recycle old dysfunctional organelles and **protein aggregates. The accumulation of such 'cellular waste' is an important feature** in cellular aging. Induction of autophagy, therefore, is a sensible anti-aging approach, which increases cellular longevity and functionality.

Autophagy plays another important role in the context of dark circles. It is a process with which keratinocytes degrade melanin-containing melanosomes. In this role, autophagy is described to be an important determinant in regulating skin color and induction of autophagy is, therefore, a sensible approach to lighten skin.

### Method

Human Keratinocytes (HaCaT) were pretreated with F111 for 24 hours. Cells were treated with 2 µM Chloroquine (inhibition of Autophagy flux). Cells were incubated for further 48 hours w/wo F111. Determination of LC3B was performed by ELISA using LC3B (D11) XP^{®} Rabbit mA (New England Biolabs, Inc.). The detection antibody was an Anti-Rabbit IgG (H+L), F(ab')2 Fragment (Alexa^{®}488) (New England Biolabs, Inc.). The fluorescence was measured at 485nm (excitation) and 535 nm (emission) in fluorescence reader (Spectramax Paradigm, Molecular Devices, LLC.).

### Results

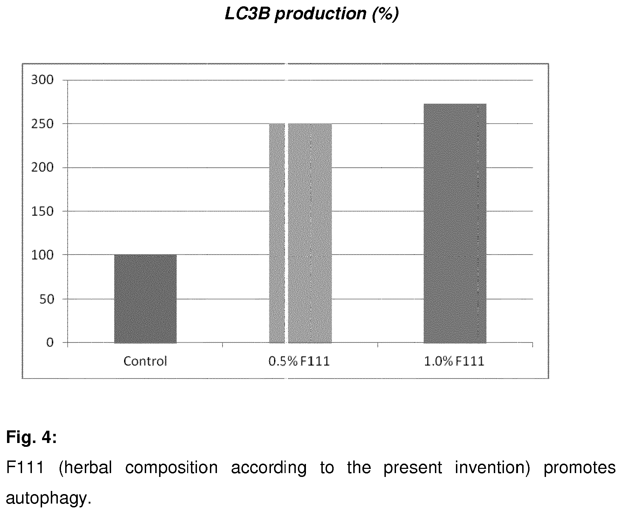

F111 at both 0.5% and 1.0% strongly induces autophagy in immortalized keratinocytes (HaCaT) (**Fig. 4**). This indicates that it strongly supports the cellular anti-aging processes and can increase cellular functionality (as proven above). With this activity F111 supports the cellular processes in breaking down melanosomes, leading to skin lightening activities.

### 5. Melanogenesis under the influence of heme and UV radiation

Pigmentation, both aging-related or consequential to inflammatory processes, plays an important role in the appearance of dark circles. It is therefore opportune to study the effect of an active ingredient on this phenomenon in more detail. Heme, being pro-inflammatory and pro-**oxidative, and playing an important role as a 'motor' behind** the processes involved in the formation of dark circles, can be considered to be an inducer of melanogenesis, specifically related to dark circles. Obviously, UV radiation is an important inducer of melanogenesis too. Like heme, UV light is an inducer of inflammatory and oxidative processes in the skin. It is therefore of interest to study whether the treatment with heme and heme together with UV radiation has an influence on melanin synthesis and if an active ingredient can reduce this.

### Method

Week 1: Human epidermal equivalents with melanocytes (epiCS-M, CellSystems GmbH) were treated for one week with 10 µM heme on the basolateral side. Some of the skin models were irradiated with 0.3J/cm² UVA + 0.03J/cm² UVB one time daily. Week 2: Heme treatment and irradiation was stopped and the models were treated (apical) with either water (control) or 3% F111 (in water) for 7 days. Melanin content of the skin models was extracted and quantified by a photometric method.

### Results

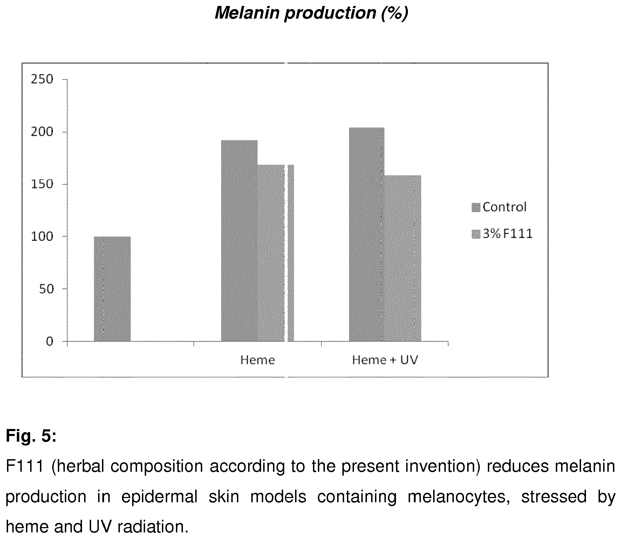

Human epidermal equivalents with melanocytes, exposed to heme, clearly show an increased production of melanin. In this setting, the treatment with F111 shows a clear reduction of melanin production (Fig. 5). Exposure of these epidermal skin equivalents to both heme and UV radiation, shows a further increase in melanin production. Here, the treatment with F111 shows a reduction of melanin production even larger than with the exposure to heme only. These results clearly demonstrate that F111 reduces melanin production under circumstances which are extremely relevant to the formation and maintenance of dark circles.

### 6. VEGF-C, lymphatic endothelial cells and keratinocytes

VEGF-C is a growth factor which promotes the production of functional lymphatic vessels. These are of eminent importance in the etiology of dark circles, as described above. Induction of the production of VEGF-C is therefore extremely important in fighting dark circles. In the eye area the accumulation of fluid takes place relatively rapidly. This fluid contains cells, ions and macromolecules, proteins specifically. The ions lead to an osmotic pressure, whereas the proteins lead to a phenomenon called oncotic pressure. Oncotic pressure can be described as a type of osmotic pressure which is induced by proteins. In an experiment where the effect of an active ingredient of VEGF-C expression is determined, osmotic pressure should therefore be taken into account. As keratinocytes play an essential role in cellular communication, including towards the lymphatic endothelial cells, it is of particular interest to analyze the effect of an active ingredient in an experimental design where the effect of keratinocyte communication to lymphatic endothelial cells, relevant to VEGF-C expression, is determined.

### Method

In an *in vitro* situation, keratinocytes release their mediators into the cell culture medium. This medium can be transferred to lymphatic endothelial cells and their ability to express VEGF-C can be determined. Human Keratinocytes (HaCaT) were pretreated with F111 for 24 hours. Osmolarity of the medium was adjusted by NaCl solution and cells were incubated for a further 24 hours.

The medium was transferred to the human dermal lymphatic endothelial cells (HDLEC), which were further incubated for 24 hours. The determination of VEGF-C was performed by ELISA (R&D Systems, Inc.; #DVEC00). Absorbance was measured ad 450 nm/540 nm. Non treated, non-damaged cells served as controls and were set to 100%.

### Results

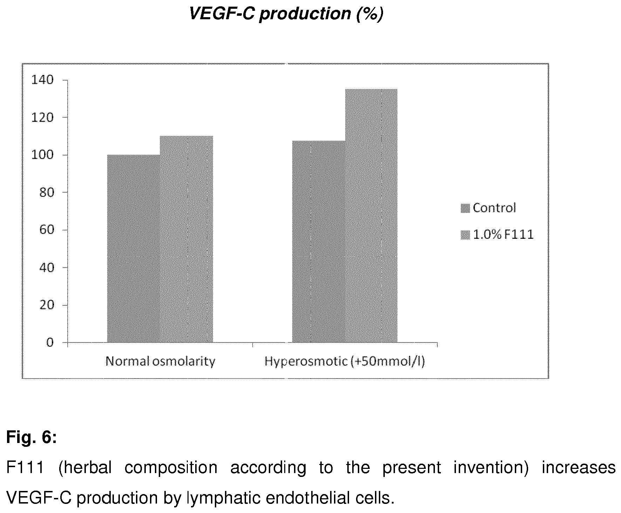

Osmotic pressure is stressful for keratinocytes. The cultivation of lymphatic endothelial cells in a medium which originates from hyperosmotically stressed keratinocytes leads to a slight increase in VEGF-C **(****Fig. 6****).** In an identical setting, now including the treatment of the keratinocytes by F111, a much clearer increase in VEGF-C production by lymphatic endothelial cells can be observed.

Cultivation of the lymphatic endothelial vessels in a medium originating from the treatment of keratinocytes at normal osmolarity and in the presence of F111, also increases VEGF-C production, albeit slightly. These results indicate that F111 supports the formation of new and functional lymph vessels. With this action, F111 plays an important role in the promotion of drainage of excess fluid, inflammatory cells and heme from the interstitial area in the dermis, an activity which is essential in fighting dark circles.

### 7. VEGF-C, TGF-β und lymphatic endothelial cells

TGF-β **is an important growth factor in the management of the dermal structure. It** induces the production of, for instance, collagens, elastin and hyaluronic acid and it plays a pivotal role in maintaining skin firmness, elasticity and thickness. Its production is reduced in aged skin, however. As the loss of dermal structure leads to an increase in skin laxity and translucency, **TGF-β** is an important factor to take into account. However, in the area under the eyes, the hyperosmotic environment caused by leakage of plasma, proteins and cells into the interstitial area, **TGF-β** has an important negative influence; it reduces the expression of VEGF-C by lymphatic endothelial cells. **TGF-β is an essential growth** factor for dermal quality, but its negative influence on VEGF-C expression under hyperosmotic pressure should be compensated for.

### Method

Human dermal lymphatic endothelial cells (HDLEC) were pretreated with F111 for 24 hours. Osmolarity of the medium was adjusted by NaCl solution. Incubation w/wo **TGF-β** commenced for another 24 hours. **The determination of VEGF-C** was performed by ELISA (R&D Systems, Inc.; #DVEC00). Absorbance was measured at 450 nm/540 nm. Non-treated, non-damaged cells served as controls and were set to 100%.

### Results

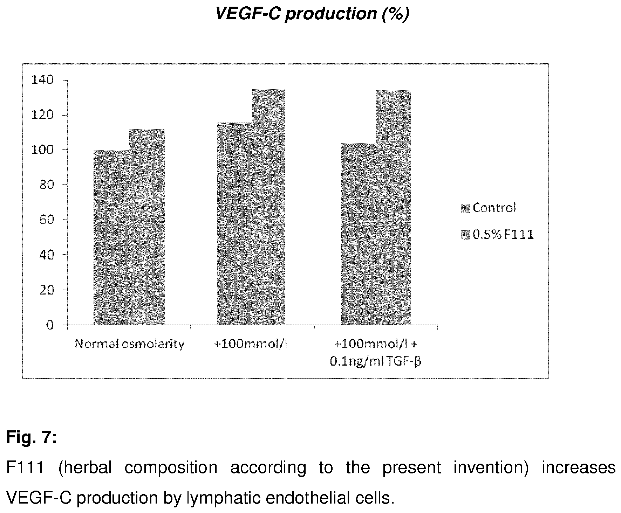

An increase in osmolarity leads to an increase in VEGF-C expression by lymphatic endothelial cells. The treatment of **TGF-β** leads to a reduction of VEGF-C expression, similar to the cells not exposed to hyperosmotic stress. **TGF-β** therefore exerts an important negative effect and prevents lymphangiogenesis. The treatment with F111 under hyperosmotic pressure leads to an increase in expression of VEGF-C. Interestingly, F111 strongly compensates for the negative effects of TGF-β (Fig. 7). The positive and relevant influence on new lymph vessel production by F111, with this, is further underlined.

### DAPI cell staining

Cell staining with DAPI (4',6-diamidino-2-phenylindol) is a measure of cell count and served for cell count correction.

DAPI is a fluorescent dye that is used in fluorescence microscopy to label DNA. The compound accumulates preferentially to AT-rich regions in the minor groove of DNA. When excited with ultraviolet light, DAPI fluoresces with blue to cyan color. In connection with double-stranded DNA, the absorption maximum is at a wavelength of 358 nm, the emission maximum at 461 nm.

Cells were grown and treated as described in the methods referred to above. A methanol solution with 0.0005% DAPI was applied to the cell layer for 15 minutes at 37°C. Subsequently, the cells were washed with double distilled water and the microtiter plate was allowed to dry for 30 minutes at 37°C.

The fluorescence was measured at 355 nm (excitation) and 600 nm (emission) in a fluorescence reader (Fluoroscan Ascent FL; Labsystems).

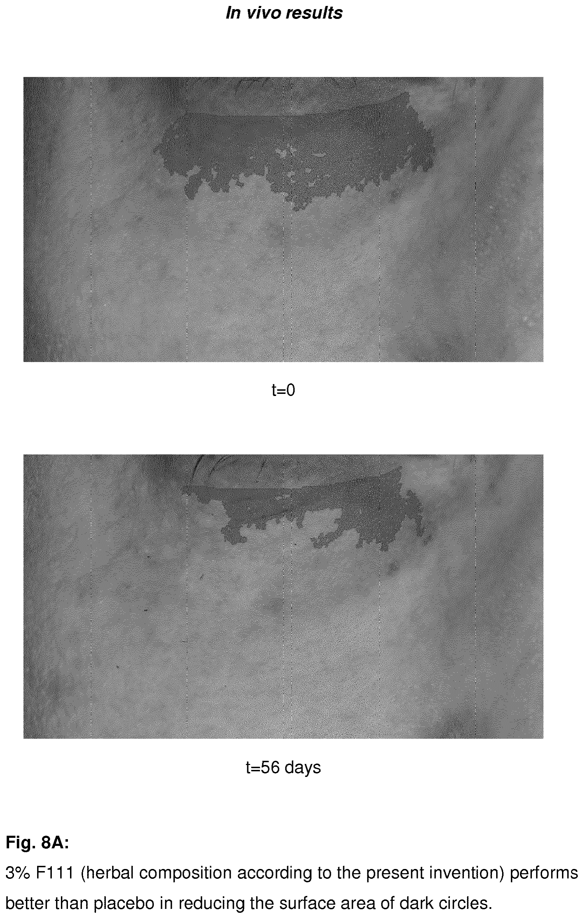

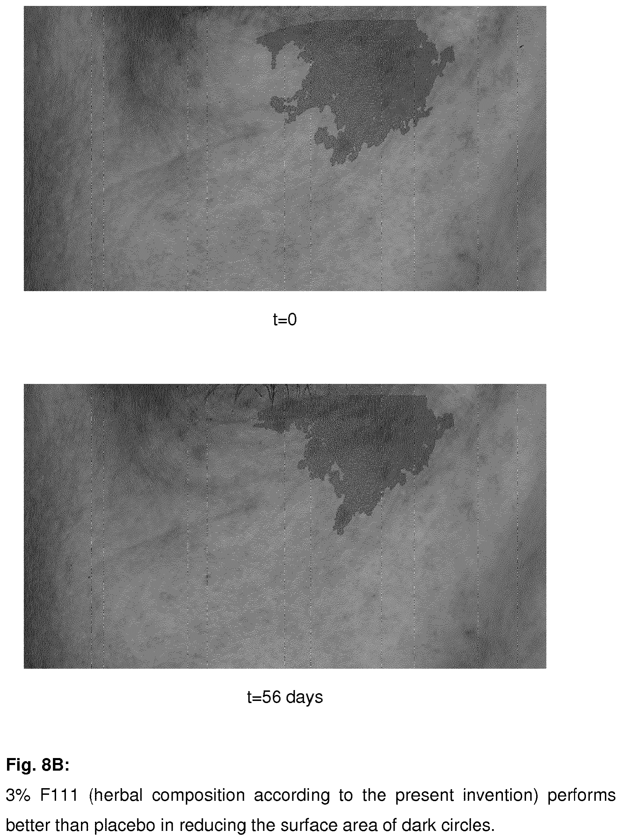

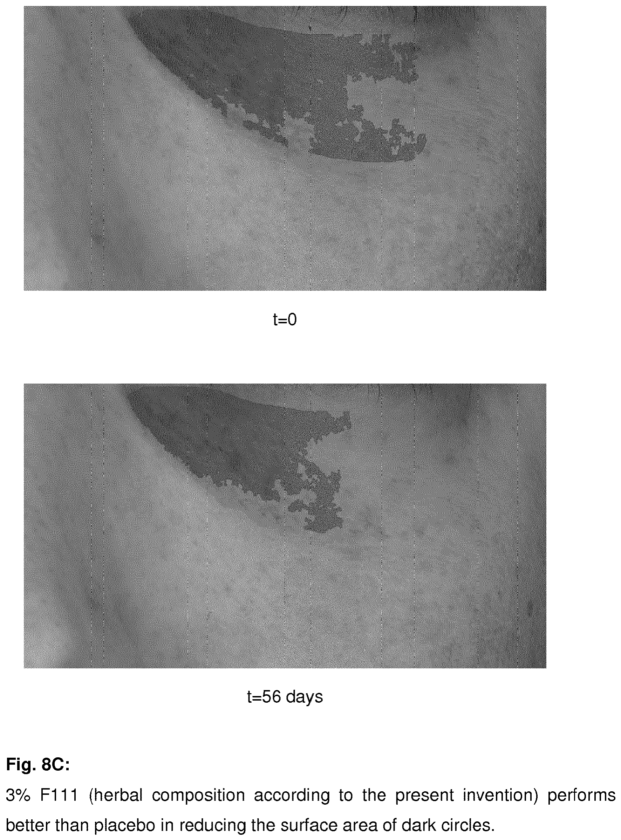

### Efficacy studies - in vivo assays (Figures 8-10)

The following studies have been carried out with a herbal composition according to the present invention, hereinafter called "F111", with preservatives
"F111" may be characterized as follows:

| *INCI Name* | *CAS No.* | *EINECS No.* |
|---|---|---|
| Hieracium Pilosella (Hawkweed) Extract (EU Name: Hieracium Pilosella Extract) | 84012-22-6 | 281-668-2 |
| Bellis Perennis (Daisy) Flower Extract (EU Name: Bellis Perennis Extract) | 84776-11-4 | 283-935-9 |
| | | |

| *Analytical Data* | | |
|---|---|---|
| Refractive index nD20 | | 1.335 - 1.345 |
| Density 20 °C | | 1.015 - 1.025 g/ml |
| pH value | | 4.0 - 5.5 |
| Dry residue (with buffer and preservatives) (2 h, 102 °C) | | 3.8 - 4.8% |
| Color value (Gardner) | | 6 - 12.5 |
| | | |
| Sodium Benzoate | | 0.40 - 0.55% |
| Sodium Dehydroacetate | | 0.10 - 0.20% |

### Reduction of dark circles

In order to assess whether F111 reduces dark circles a study was performed where 12 volunteers applied cosmetic formulations, one containing 3% F111 and a corresponding placebo, for 56 days, twice daily on each of the eye areas. At the beginning of the study and after 56 days of applying the cosmetic products, standardized photographs were taken by the use of a VISIA^{®} device (Canfield Scientific, Inc.). This device allows for detailed imaging in a standardized environment, disallowing external light influences. This allows for the comparison of different pictures of the same person at different time points.

The standardized photographs were then digitally analyzed by Newtone Technologies (Lyon, France). By making use of the combined technologies offered by Canfield and Newtone conclusions can be drawn on the effect of a cosmetic product on dark circles.

Through the digital analysis of the photographs important parameters relevant to dark circles are generated, for instance, the so-called ITA° (Individual Typological Angle). The ITA° is a parameter relevant for darkness of skin. Overall a reduction of dark circles is obtained when ITA° increases in value.

An essential feature in the reduction of dark circles is their surface area. A reduction of the surface area of dark circles plays an extraordinarily important role in the visibility of dark circles.

### Results

Individual results show that the application of 3% F111 for 56 days clearly leads to a reduction of the surface area of dark circles (Fig. 8)

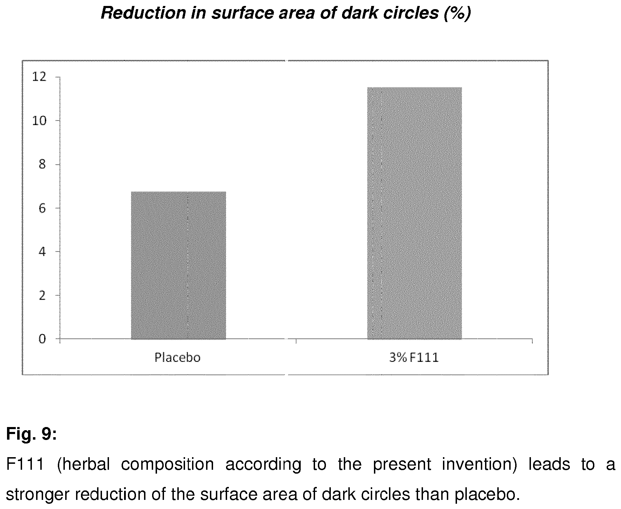

An analysis of the effects obtained with 3% F111 and placebo formulation, now with a focus on the actual surface area of the dark circles on all individuals (Fig. 9), clearly show that 3% F111 reduces the surface area of the dark circles more effectively than placebo.

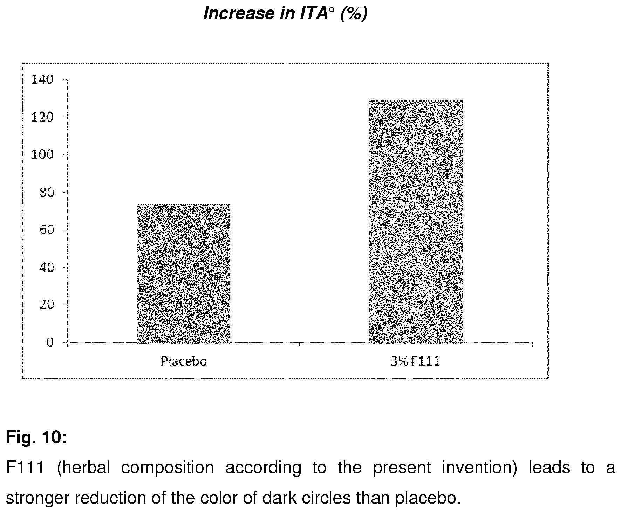

To further underline the effects of F111 on dark circles an additional analysis was made on the parameters relevant to their color and visibility. The application of a formulation containing 3% F111 leads to an increase in value of the parameter ITA°, clearly larger than obtained with placebo (Fig. 10).

### COSMESTIC OR PHARMACEUTICAL FORMULATIONS OF THE INVENTION

The compositions comprising the mixtures of components of said plants (herbal compositions) according to the present invention can be administered by any means which causes contact between said mixtures and the site of action in a mammal's body, preferably being that of a human being, and the form of a cosmetic or pharmaceutical formulation which contains them.

According to the present invention, cosmetic and pharmaceutical formulations for topical application and the topical application of the formulations according to the present invention are particularly preferred.

Thus, the present invention further provides pharmaceutical and cosmetic formulations suitable for topical/dermatological application (around the area of the human eye) comprising the (herbal) compositions according to the present invention.

The (herbal) compositions according to the present invention may be included in common cosmetic and pharmaceutical formulations known to the person of ordinary skills in the art (see, e.g., Bauer et al., Pharmazeutische Technologie, 5. Edt. Govi-Verlag Frankfurt, 1997; Rudolf Voigt, Pharmazeutische Technologie, 9. Edt., Deutscher Apotheker Verlag Stuttgart, 2000), such as O/W and W/O creams, O/W and W/O emulsions (milk), ointments, tonic (lotion), gels, multiple emulsions (W/O/W and O/W/O), cosmetic dispersions (hydrodispersions and lipodispersions), sticks, formulations comprising a tenside, in particular a surfactant cleanser, simple solutions (oily or aqueous), a micellar water or a mask.

The cosmetic or pharmaceutical formulation according to the present invention preferably is an emulsified base, in particular O/W creams and O/W emulsions or a gel, in particular a hydrogel, and is particularly suitable for treatment of the skin under the eyes.

The herbal compositions of the present invention may also be incorporated into cosmetic or pharmaceutical delivery systems and/or sustained release systems.

Examples of delivery systems or sustained release systems include, without limitation, liposomes, mixed liposomes, oleosomes, nicososmes, ethosomes, milliparticles, microparticles, nanoparticles and solid lipid nanoparticles, nanostructured lipid carriers, sponges, cyclodextrins, vesicles, micelles, mixed micelles of surfactants, surfactant-phospholipid mixed micelles, millispheres, microspheres, nanospheres, lipospheres, millicacpsules, microcapsules, nanocapsules as well as microemulsions and nanoemulsions.

The compositions/herbal compositions according to the present invention can also be incorporated in/absorbed to fabric, non-woven fabric or tissue or cotton wool balls or pads and may thus be, e.g., applied in form of a moist tissue pad or moist tissue mask.

The formulations according to the present invention may also be packaged in soft gelatine capsules or sachets for single use dosage units.

The cosmetically or/and pharmaceutically effective amount of the herbal compositions of the invention which should be administered, as well as their dosage, will depend on numerous factors, including age, state of the patient, the nature or severity of the condition, disorder or disease to be treated or cared for, the route an frequency of administration and of the particular nature of the herbal composition to be used.

Cosmetically or/and pharmaceutically effective amount is understood to mean a nontoxic but sufficient amount of the herbal composition to provide the desired effect.

The herbal compositions of the present invention are used in the cosmetic or pharmaceutical formulation of the invention at cosmetically or pharmaceutically effective concentrations to achieve the desired effect.

As a rule the topical formulations according to the present inventions may comprise 1 to 50% (w/w) with regard to the total weight of the formulation of the (herbal) composition according to the present invention.

In patch tests concentrations up to 50% (w/w) have not shown to elicit any skin irritation.

For cosmetic formulations a concentration of about 1 to 10% (w/w), 1 to 5% (w/w), 3 to 5% (w/w), 5% (w/w) or 3% (w/w) of the mixture of the components of *Bellis perennis* (and *Hieracium pilosella)* (the herbal composition) is preferred. A concentration of 3-5% (w/w) is particularly preferred.

In particular, pharmaceutical formulations may comprise a higher concentration of 1 to 30%, 10 to 30% but may also preferably comprise a concentration of about 1 to 10% (w/w), 1 to 5% (w/w), 3 to 5% (w/w), 5% (w/w) or 3% (w/w) of the mixture of the components of *Bellis perennis* (and *Hieracium pilosella)* (the herbal composition).

The cosmetic and pharmaceutical formulations according to the present invention, in addition to the usual excipients well known to a person of skill in the art, may comprise additional ingredients such as:

### Ingredients for enhancing firmness and elasticity

The agents for enhancing firmness and elasticity of the skin and for minimizing under eye circles can be selected from the group consisting of collagen inducers and silicon derivatives. Preferred are collagen inducers such as milk peptide complex (MPC) at a concentration range of about 0.2 to about 2.0%, preferably at a concentration at about 0.5 to 1.9%. Further preferred is a silicon derivative that comprises a complex of methylsilanol elastinate and methylsilanol aspartate hydroxyprolinate. The concentration of this complex in the emulsified cosmetic composition can range from 3.4% to 4.6%. Four per cent is the preferred concentration. The preferred respective weight ratios of methylsilanol elastinate and methylsilanol asparatate hydroxyprolinate in the complex are 25% and 75%. A commercial source of methylsilanol elastinate and methylsilanol aspartate hydroxyprolinate is Exsymol S.A.M. (Monte Carol, Monaco). Methylsilanol elastinate is sold under the trade name of "Proteosilane-C" and methylsilanol aspartate hydroxyprolinate is sold under the trade name of "Hydroxyprolisilane-C.

### Ingredients for reducing effects of free-radicals

The anti-free radical ingredients of an emulsified cosmetic composition can be selected form the group consisting of derivatives of vitamins C or **E,** selenium metal compounds, or beta carotene derivatives.

### Preservatives

Preservatives are used to prevent the growth of microbes. A sufficient quantity of one or more preservatives thus may be added. The topical formulations according to the present invention may thus contain one or more preservatives commonly known in the art such as sodium dehydroacetate, 2-hydroxybenzoic acid, 1-phenyl-1-propanol, Dermosoft^{®} 700B (mixture of levulinic acid; sodium levulinate; glycerin and water), phenethyl alcohol or sodium benzoate at concentrations commonly used in the art.

### Antioxidants

To maintain the composition's color and prevent malodorous developments, an antioxidant may be included in the cosmetic and pharmaceutical compositions. The antioxidant can be an ascorbyl ester selected from the group consisting of ascorbyl palmitate, ascorbyl myristate, and ascorbyl stearate. A preferred antioxidant is ascorbyl palmitate.

### Emulsifiers

Emulsifiers serve two functions. They act like a solubilizing agent to combine the water soluble and non-water soluble phases together; that is, form a stable bridge between the waters and the oils of the ingredients. The emulsifiers also serve as emollients, providing a pleasant, aesthetically appropriate, tactile feeling when the emulsified composition is applied to the skin. These can be propylene glycol isoceteth-3 actetate and laureth-2 benzoate. One of the emulsifiers can be an ester of an unsaturated fatty acid selected from the group consisting of of isodecyl oleate, isononyl oleate, isoundecyl oleate, isononyl elaidate, isodecyl elaidate, isoundecyl elaidate, isononyl vaccenate, isodecyl vaccenate, and isoundecyl vaccenate. A preferred ester of an unsaturated fatty acid is isodecyl oleate.

Another emulsifier can be an ester of a short-chain saturated fatty acid selected from the group consisting of myristyl octanoate, myristyl heptanoate, myristyl nonanoate, lauryl heptanoate, lauryl octanoate, lauryl nonanoate, palmityl heptanoate, palmityl octanoate, and palmityl nonanoate. A typical ester of a short-chain saturated fatty acid for emollient use in the emulsified cosmetic composition can be myristyl octanoate.

A preferred emulsifier can be a glyceryl ester selected from the group consisting of glyceryl stearate, glyceryl palmitate, and glyceryl arachidate. A typical glyceryl ester for use in the emulsified cosmetic composition is glyceryl stearate. Other preferred emulsifiers include triethanolamine and 3-cyclohexene-1-methanol, alpha, 4-dimethyl-alpha-(4-methyl-3-pentenyl) ("bisabolol").

### Pigment

The cosmetic or pharmaceutical composition can include at least one pigment for coloration, preferably red iron oxide

The formulation examples below are included for illustrative purposes only and shall not limit the scope of the invention.

### FORMULATION EXAMPLES

### Example 1

### Lightening & Decongestant Eye Mask (Rinse Off)

Mix A and stir until completely dispersed and soaked. Add B under stirring and adjust pH value to 5.5 with C.

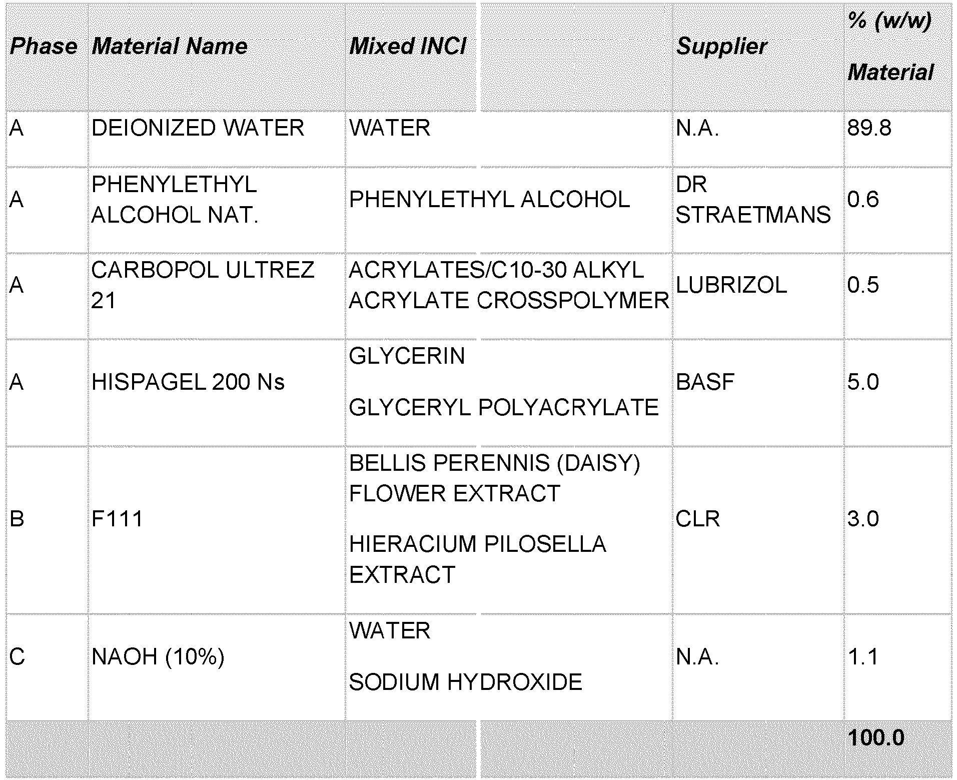

### Example 2

### Anti Dark Circle Eye Lotion

Mix A in the given order and stir until uniform slowly. Add B and to A while stirring. Add C and stir until homogenous.

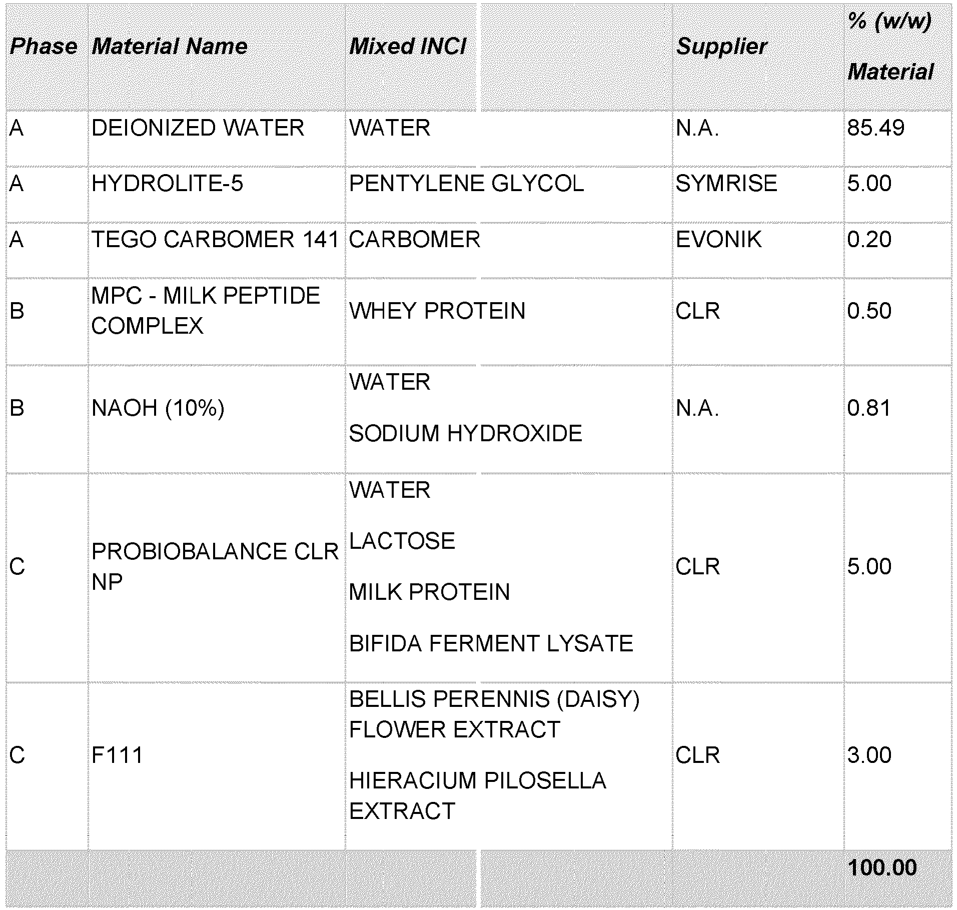

### Example 3

### Brightening Facial Cream (O/W)

Add A and stir until completely dispersed. Mix B, heat up A and B to 75°C-80°C separately. Add B to A while stirring. Homogenize for 2 minutes with Ultra Turrax. Cool down while stirring and add C at room temperature. Adjust pH value to 5.0 with D as desired.

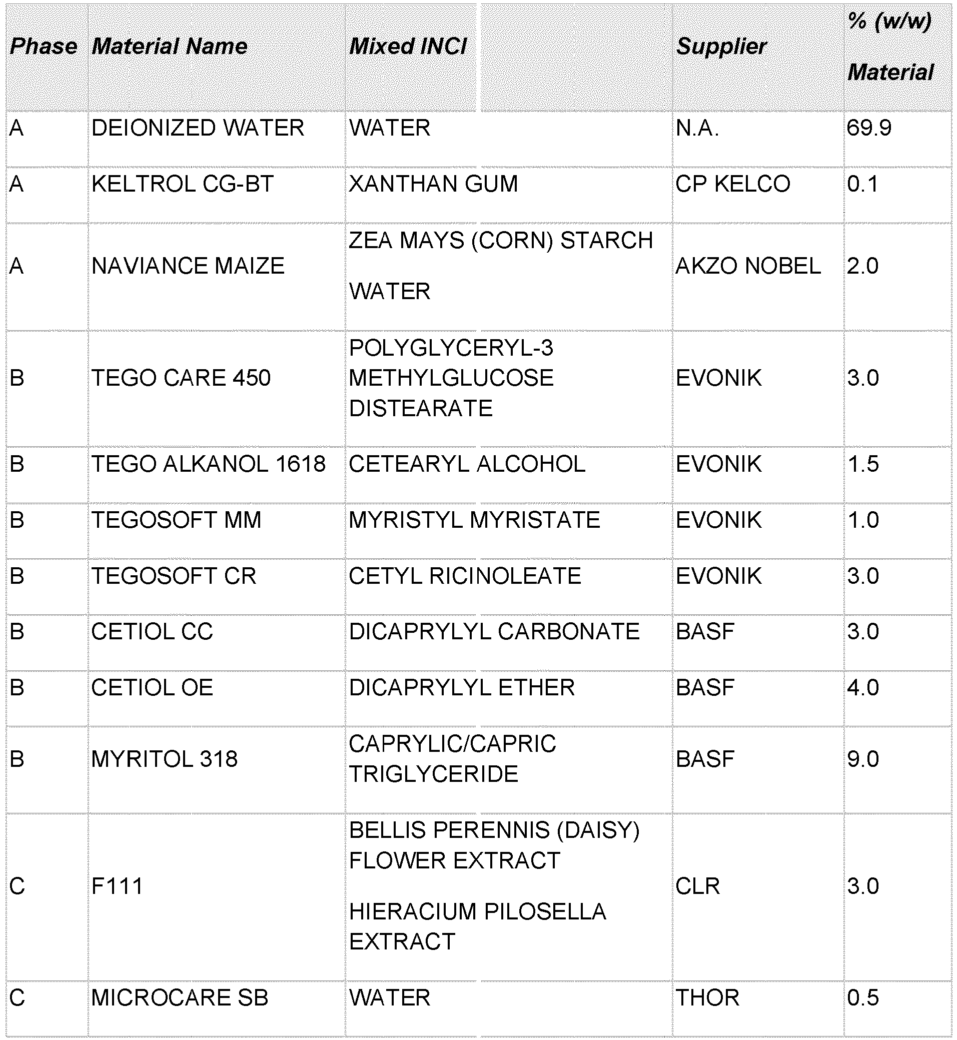

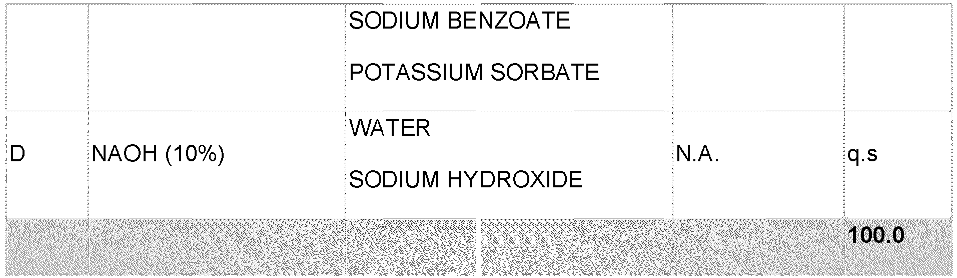

### Example 4

### Night Lifting Eye Care (Leave On)

Mix A and stir until completely soaked. Add B and stir for a short. Add C and stir until uniform. Adjust pH value to 6-6.5 with D.

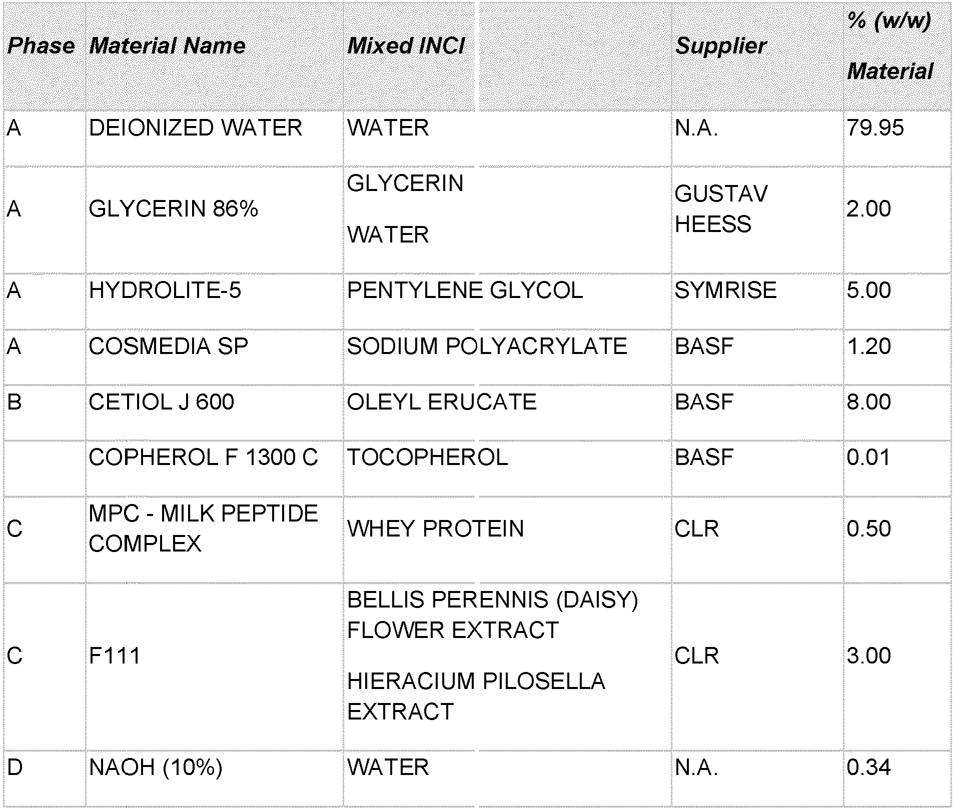

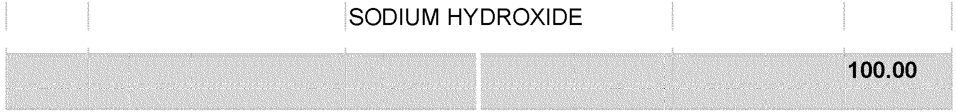

## Claims

1. A herbal composition comprising a mixture of components obtained from *Bellis perennis* and *Hieracium pilosella,* whereby the components are prepared by separate or combined preparation, wherein the components are obtained by extraction with a citrate buffer pH 5.0.

2. Herbal composition according to claim 1, wherein the components are obtained by decoction, digestion, percolation, soxhletation , maceration jointly or separately.

3. Herbal composition according to any one of claims 1 to 2, wherein the components are obtained by extraction with a sodium citrate buffer according to Sörensen with 0.1 M citric acid monohydrate (C₆H₈O₇xH₂O) and 0.1 M trisodium citrate dihydrate solution having a pH of 5.0.

4. Herbal composition according to any one of claims 1 to 3, wherein the mixture of components obtained from *Bellis perennis* and *Hieracium pilosella* are present in a ratio of 1:1 to 9:1, 1:1 to 3:1, 1:1 to 5:2, 1:1 to 2:1 or 1:1 to 3:1 (w:w).

5. Herbal composition according to claim 4, wherein the mixture of components obtained from *Bellis perennis* and *Hieracium pilosella* are present in a ratio of 1:1 or 3:1.

6. Herbal composition according to any of claims 1 to 5,
wherein the components are obtained by separate extraction of the dried flower heads of *Bellis perennis* and the dried aerial parts of *Hieracium pilosella* with a sodium citrate buffer according to Sörensen with 0.1 M citric acid monohydrate (C₆H₈O₇xH₂O) and 0.1 M trisodium citrate dihydrate solution having a pH of 5.0.

7. Herbal composition according to any of claims 1 to 6, wherein the proportion of drug and extraction medium ranges from 5:100 to 7:100.

8. Herbal composition according to any of claims 1 to 7,
wherein the components of *Bellis perennis* are obtained by extracting *Bellis perennis* with citrate buffer pH 5.0 at a temperature of 60°C for 4 to 8 hours, whereby the proportion of drug:extraction medium is 7:100 (7%) to 10:100 (10%) and the plant material is separated from the liquid phase, and
the components of *Hieracium pilosella* are obtained by extracting *Hieracium pilosella* with citrate buffer pH 5.0 at a temperature of 25°C to 60°C for 2 to 4 hours, whereby the proportion of drug: extraction medium is 5:100 (5%) to 7:100 (7%) followed by fractionating the crude extract by ultrafiltration (MW cut-off: 100 kDa) to obtain the < 100 kDa fraction of *Hieracium pilosella,* and
combining the two separately obtained extracts.

9. Herbal composition according to claim 8, wherein the components of *Bellis perennis* are obtained by extracting dried flower heads of *Bellis perennis* and the components of *Hieracium pilosella* are obtained by extracting the dried whole aerial part of the plant.

10. Herbal composition according to any of claims 1 to 9, wherein the mixture of components obtained by separate extraction of *Bellis perennis* and *Hieracium pilosella* is present in a ratio in the range of 1:1 to 3:1.

11. A herbal composition according to any one of claims 1 to 10, comprising a mixture of components obtained from *Bellis perennis* and *Hieracium pilosella,* wherein the composition comprises chlorogenic acid, cyanidin 3-malonylglucoside, apigeninhexoside, apigeninglucuronide, apigeninmethylglucuronide, kaempferolhexoside, quercetinhexoside, isorhamnetinhexoside, apigeninglucoside, isorhamnetinglucuronide, isorhamnetinglucoside, isorhamnetin, perennisoside III, IV, V, VI, perennisoside XII, perennisoside XIII, perennisoside XIV, perennisoside XV, perennisoside XVIII & XIX, desacyl-perennisoside I, II & III, desacyl-perennisoside VIII & IX and desacyl-perennisoside X & XI.

12. Herbal composition according to claim 11, further optionally comprising one or more of the saponine compounds bellisisosides C & E, bellisisoside D, perennisosides I & II, perennisoside VII, perennisosides VIII & IX, perennisosides X & XI, perennisosides XVI, perennisaponine A and perennisaponines G; H.

13. Herbal composition according to any one of claims 1 to 12, having a pH-value of between about pH 4.0 to 6.5.

14. Herbal composition according to any of claims 1 to 13, further comprising a preservative selected from sodium dehydroacetate, 2-hydroxybenzoic acid, 1-phenyl-1-propanol, Dermosoft^{®} 700B, phenethyl alcohol and sodium benzoate.

15. Pharmaceutical or cosmetic formulation, comprising a herbal composition according to any one of claims 1 to 14.

16. Cosmetic formulation according to claim 15.

17. Pharmaceutical formulation comprising a herbal composition according to any one of claims 1 to 14 for use in the therapeutic treatment or prevention of periorbital dark circles, and/or periorbital edema, wherein the pharmaceutical formulation acts as an autophagy stimulator, heme oxygenase activator, VEGF-C production trigger and/or melanin synthesis reducer.

18. Pharmaceutical formulation for use according to claim 17, wherein the dark circles under the eyes and/or puffiness are caused by any one of heredity, allergies, eczema (atopic dermatitis), contact dermatitis, hay fever/allergic rhinitis, iron deficiency or other iron disorders, minor trauma, crying, fluid retention, excessive exposure to the sun, rubbing and scratching of eyes, and medication.

19. Non-therapeutic use of a herbal composition according to any one of claims 1 to 14 as a autophagy stimulator, heme oxygenase activator, VEGF-C production trigger and/or melanin synthesis reducer for the cosmetic treatment or prevention of periorbital dark circles and/or puffy eyes.

20. Use according to claim 19, wherein the dark circles under the eyes and/or puffiness are caused by any one of thinning skin and/or loss of fat and collagen with age (ageing), life style choices, sleep deprivation/fatigue, oversleeping, fluid retention, and influence of circadian rhythm by life style and life conditions.

21. Pharmaceutical formulation according to any one of claims 15 to 18, comprising a herbal composition according to any one of claims 1 to 12 at a concentration of 3 - 5% (w/w) of the total weight of the total formulation.

22. Pharmaceutical or cosmetic formulation according to any one of claims 15 to 21 for topical application.

23. Pharmaceutical or cosmetic formulation according to claim 22, wherein said formulation is a cream, an ointment, an emulsion (milk), a tonic (lotion), stick, dispersion, a formulation comprising a surfactant cleanser, a solution, a micellar water, a gel, a mask, a moist tissue pad or moist tissue mask.

24. Use according to any one of claims 19 to 20 for topical application.

25. Use according to claim 24 for topical application as a cream, an ointment, an emulsion (milk), a tonic (lotion), stick, dispersion, a formulation comprising a surfactant cleanser, a solution, a micellar water, a gel, a mask, a moist tissue pad or moist tissue mask.

## Patentansprüche

1. Eine pflanzliche Zusammensetzung, umfassend ein Gemisch aus Bestandteilen, erhalten aus *Bellis perennis* und *Hieracium pilosella,* wobei die Bestandteile durch getrennte oder kombinierte Aufbereitung hergestellt werden, wobei die Bestandteile durch Extraktion mit einem Citratpuffer pH 5,0 erhalten werden.

2. Pflanzliche Zusammensetzung gemäß Anspruch 1, wobei die Bestandteile durch Decoction, Digestion, Perkolation, Soxhletextraktion, Mazeration gemeinsam oder getrennt voneinander erhalten werden.

3. Pflanzliche Zusammensetzung gemäß einem der Ansprüche 1 bis 2, wobei die Bestandteile durch Extraktion mit einem Natriumcitratpuffer gemäß Sörensen mit 0,1 M Citronensäuremonohydrat (C₆H₈O₇xH₂O) und 0,1 M Trinatriumcitratdihydrat-Lösung mit einem pH-Wert von 5,0 erhalten werden.

4. Pflanzliche Zusammensetzung gemäß einem der Ansprüche 1 bis 3, wobei das Gemisch aus den aus *Bellis perennis* und *Hieracium pilosella* erhaltenen Bestandteilen in einem Verhältnis von 1:1 bis 9:1, 1:1 bis 3:1, 1:1 bis 5:2, 1:1 bis 2:1 oder 1:1 bis 3:1 (Gew./Gew.) vorliegt.

5. Pflanzliche Zusammensetzung gemäß Anspruch 4, wobei das Gemisch aus den aus *Bellis perennis* und *Hieracium pilosella* erhaltenen Bestandteilen in einem Verhältnis von 1:1 oder 3:1 vorliegt.

6. Pflanzliche Zusammensetzung gemäß einem der Ansprüche 1 bis 5,
wobei die Bestandteile durch separate Extraktion der getrockneten Blütenstände von *Bellis perennis* und der getrockneten oberirdischen Pflanzenstände von *Hieracium pilosella* mit einem Natriumcitratpuffer gemäß Sörensen mit 0,1 M Citronensäuremonohydrat (C₆H₈O₇xH₂O) und 0,1 M Trinatriumcitratdihydrat-Lösung mit einem pH-Wert von 5,0 erhalten werden.

7. Pflanzliche Zusammensetzung gemäß einem der Ansprüche 1 bis 6, wobei das Verhältnis von Arzneidroge und Extraktionsmittel im Bereich von 5:100 bis 7:100 liegt.

8. Pflanzliche Zusammensetzung gemäß einem der Ansprüche 1 bis 7,
wobei die Bestandteile von *Bellis perennis* durch die Extraktion von *Bellis perennis* mit einem Citratpuffer pH 5,0 bei einer Temperatur von 60°C für 4 bis 8 Stunden erhalten werden, wobei das Verhältnis von Arzneidroge:Extraktionsmittel 7:100 (7%) bis 10:100 (10%) beträgt und das Pflanzenmaterial von der flüssigen Phase getrennt wird, und die Bestandteile von *Hieracium pilosella* durch Extraktion von
*Hieracium pilosella* mit einem Citratpuffer pH 5,0 bei einer Temperatur von 25°C bis 60°C für 2 bis 4 Stunden erhalten wird, wobei das Verhältnis von Arzneidroge:Extraktionsmittel 5:100 (5%) bis 7:100 (7%) beträgt, gefolgt von einer Fraktionierung des Rohextrakts durch Ultrafiltration (MW-Cut Off: 100 kDa), um die < 100 kDa-Fraktion von *Hieracium pilosella* zu erhalten, und
Kombinieren der zwei getrennt voneinander erhaltenen Extrakte.

9. Pflanzliche Zusammensetzung gemäß Anspruch 8, wobei die Bestandteile von *Bellis perennis* durch Extraktion von getrockneten Blütenständen von *Bellis perennis* erhalten werden und die Bestandteile von *Hieracium pilosella* durch Extraktion des getrockneten gesamten oberirdischen Pflanzenstands der Pflanze erhalten werden.

10. Pflanzliche Zusammensetzung gemäß einem der Ansprüche 1 bis 9, wobei das Gemisch aus den durch getrennte Extraktion von *Bellis perennis* und *Hieracium pilosella* erhaltenen Bestandteilen in einem Verhältnis im Bereich von 1:1 bis 3:1 vorliegt.

11. Eine pflanzliche Zusammensetzung gemäß einem der Ansprüche 1 bis 10, umfassend ein Gemisch aus Bestandteilen erhalten aus *Bellis perennis* und *Hieracium pilosella,*
wobei die Zusammensetzung Chlorogensäure, Cyanidin-3-malonylglucosid, Apigeninhexosid, Apigeninglucuronid, Apigeninmethylglucuronid, Kaempferolhexosid, Quercetinhexosid, Isorhamnetinhexosid, Apigeninglucosid, Isorhamnetinglucuronid, Isorhamnetinglucosid, Isorhamnetin, Perennisosid III, IV, V, VI, Perennisosid XII, Perennisosid XIII, Perennisosid XIV, Perennisosid XV, Perennisosid XVIII & XIX, Desacyl-perennisosid I, II & III, Desacyl-perennisosid VIII & IX und Desacyl-perennisosid X & XI umfasst.

12. Pflanzliche Zusammensetzung gemäß Anspruch 11, gegebenenfalls ferner umfassend eine oder mehrere der Saponinverbindungen Bellisisoside C & E, Bellisisosid D, Perennisoside I & II, Perennisosid VII, Perennisoside VIII & IX, Perennisoside X & XI, Perennisosid XVI, Perennisaponin A und Perennisaponine G; H.

13. Pflanzliche Zusammensetzung gemäß einem der Ansprüche 1 bis 12 mit einem pH-Wert zwischen etwa 4,0 bis 6,5.

14. Pflanzliche Zusammensetzung gemäß einem der Ansprüche 1 bis 13, ferner umfassend ein Konservierungsmittel, ausgewählt aus Natriumdehydroacetat, 2-Hydroxybenzoesäure, 1-Phenyl-1-propanol, Dermosoft^{®} 700B, Phenethylalkohol und Natriumbenzoat.

15. Pharmazeutische oder kosmetische Formulierung, umfassend eine pflanzliche Zusammensetzung gemäß einem der Ansprüche 1 bis 14.

16. Kosmetische Formulierung gemäß Anspruch 15.

17. Pharmazeutische Formulierung, umfassend eine pflanzliche Zusammensetzung gemäß einem der Ansprüche 1 bis 14 zur Verwendung bei der therapeutischen Behandlung oder Vorbeugung von dunklen Augenringen und/oder periorbitalem Ödem, wobei die pharmazeutische Formulierung als ein Autophagie-Stimulator, Häm-Oxygenase-Aktivator, VEGF-C-Produktionsauslöser und/oder Melaninsynthese-Reduktionsmittel wirkt.

18. Pharmazeutische Formulierung zur Verwendung gemäß Anspruch 17, wobei die dunklen Ringe unter den Augen und/oder Schwellungen durch eine der folgenden Ursachen hervorgerufen werden: Vererbung, Allergien, Ekzem (atopische Dermatitis), Kontaktdermatitis, Heuschnupfen/allergische Rhinitis, Stress, Eisenmangel oder andere Eisenstoffwechselstörungen, kleinere Traumata, Weinen, Flüssigkeitsansammlungen, übermäßige Sonneneinstrahlung, Reiben und Kratzen der Augen sowie Medikamente.

19. Nicht-therapeutische Verwendung einer pflanzlichen Zusammensetzung gemäß einem der Ansprüche 1 bis 14 als ein Autophagie-Stimulator, Häm-Oxygenase-Aktivator, VEGF-C-Produktionsauslöser und/oder Melaninsynthese-Reduktionsmittel zur kosmetischen Behandlung oder Vorbeugung von dunklen Augenringen und/oder geschwollenen Augen.

20. Verwendung gemäß Anspruch 19, wobei die dunklen Ringe unter den Augen und/oder Schwellungen durch eine der folgenden Ursachen hervorgerufen werden: dünner werdende Haut und/oder Verlust von Fett und Kollagen mit zunehmendem Alter (Alterung), Lebensweise, Schlafmangel/Müdigkeit, zu viel Schlaf, Flüssigkeitsansammlungen und Einfluss des Biorhythmus durch Lebensweise und Lebensbedingungen.

21. Pharmazeutische Formulierung gemäß einem der Ansprüche 15 bis 18, umfassend eine pflanzliche Zusammensetzung gemäß einem der Ansprüche 1 bis 12 in einer Konzentration von 3 - 5% (Gew./Gew.) des Gesamtgewichts der gesamten Formulierung.

22. Pharmazeutische oder kosmetische Formulierung gemäß einem der Ansprüche 15 bis 21 zur topischen Anwendung.

23. Pharmazeutische oder kosmetische Formulierung gemäß Anspruch 22, wobei die Formulierung eine Creme, eine Salbe, eine Emulsion (Milch), ein Tonikum (Lotion), ein Stift, eine Dispersion, eine Formulierung umfassend ein grenzflächenaktives Reinigungsmittel, eine Lösung, ein Mizellenwasser, ein Gel, eine Maske, ein feuchtes Gewebepad oder eine feuchte Gewebemaske ist.

24. Verwendung gemäß einem der Ansprüche 19 bis 20 zur topischen Anwendung.

25. Verwendung gemäß Anspruch 24 zur topischen Anwendung als eine Creme, eine Salbe, eine Emulsion (Milch), ein Tonikum (Lotion), ein Stift, eine Dispersion, eine Formulierung umfassend ein grenzflächenaktives Reinigungsmittel, eine Lösung, ein Mizellenwasser, ein Gel, eine Maske, ein feuchtes Gewebepad oder eine feuchte Gewebemaske.

## Revendications

1. Composition à base de plantes comprenant un mélange de composants obtenus à partir de *Bellis perennis* et de *Hieracium pilosella,* dans laquelle les composants sont préparés par préparation séparée ou combinée, dans laquelle les composants sont obtenus par extraction avec un tampon citrate à pH 5,0.

2. Composition à base de plantes selon la revendication 1, dans laquelle les composants sont obtenus par décoction, digestion, percolation, extraction Soxhlet, macération conjointement ou séparément.

3. Composition à base de plantes selon la revendication 1 ou 2, dans laquelle les composants sont obtenus par extraction avec un tampon citrate de sodium conformément à Sörensen avec de l'acide citrique monohydraté (C₆H₈O₇xH₂O) 0,1 M et une solution 0,1 M de citrate trisodique dihydraté ayant un pH de 5,0.

4. Composition à base de plantes selon l'une quelconque des revendications 1 à 3, dans laquelle le mélange de composants obtenus à partir de *Bellis perennis* et de *Hieracium pilosella* est présent en un rapport de 1/1 à 9/1, 1/1 à 3/1, 1/1 à 5/2, 1/1 à 2/1 ou 1/1 à 3/1 (en poids/poids).

5. Composition à base de plantes selon la revendication 4, dans laquelle le mélange de composants obtenus à partir de *Bellis perennis* et de *Hieracium pilosella* est présent en un rapport de 1/1 ou 3/1.

6. Composition à base de plantes selon l'une quelconque des revendications 1 à 5, dans laquelle les composants sont obtenus par extraction séparée des capitules séchés de *Bellis perennis* et des parties aériennes séchées de *Hieracium pilosella* avec un tampon citrate de sodium conformément à Sörensen avec de l'acide citrique monohydraté (C₆H₈O₇xH₂O) 0,1 M et une solution 0,1 M de citrate trisodique dihydraté ayant un pH de 5,0.

7. Composition à base de plantes selon l'une quelconque des revendications 1 à 6, dans laquelle la proportion de médicament et de milieu d'extraction est située dans la plage allant de 5/100 à 7/100.

8. Composition à base de plantes selon l'une quelconque des revendications 1 à 7, dans laquelle les composants de *Bellis perennis* sont obtenus par extraction de *Bellis perennis* avec du tampon citrate à pH 5,0 et à une température de 60 °C pendant 4 à 8 heures, moyennant quoi la proportion médicament/milieu d'extraction est de 7/100 (7 %) à 10/100 (10 %) et le matériau végétal est séparé de la phase liquide, et les composants de *Hieracium pilosella* sont obtenus par extraction de *Hieracium pilosella* avec du tampon citrate à pH 5,0 et à une température de 25 °C à 60 °C pendant 2 à 4 heures, moyennant quoi la proportion médicament/milieu d'extraction est de 5/100 (5 %) à 7/100 (7 %), suivie d'un fractionnement de l'extrait brut par ultrafiltration (seuil des PM : 100 kDa) pour que soit obtenue la fraction < 100 kDa de *Hieracium pilosella,* et combinaison des deux extraits obtenus séparément.

9. Composition à base de plantes selon la revendication 8, dans laquelle les composants de *Bellis perennis* sont obtenus par extraction de capitules séchés de *Bellis perennis* et les composants de *Hieracium pilosella* sont obtenus par extraction de la partie aérienne entière séchée de la plante.

10. Composition à base de plantes selon l'une quelconque des revendications 1 à 9, dans laquelle le mélange de composants obtenus par extraction séparée de *Bellis perennis* et de *Hieracium pilosella* est présent en un rapport situé dans la plage allant de 1/1 à 3/1.

11. Composition à base de plantes selon l'une quelconque des revendications 1 à 10, comprenant un mélange de composants obtenus à partir de *Bellis perennis* et de *Hieracium pilosella,* laquelle composition comprend de l'acide chlorogénique, du 3-malonylglucoside de cyanidine, de l'hexoside d'apigénine, du glucuronide d'apigénine, du méthylglucuronide d'apigénine, de l'hexoside de kaempférol, de l'hexoside de quercétine, de l'hexoside d'isorhamnétine, du glucoside d'apigénine, du glucuronide d'isorhamnétine, du glucoside d'isorhamnétine, de l'isorhamnétine, du pérennisoside III, IV, V, VI, du pérennisoside XII, du pérennisoside XIII, du pérennisoside XIV, du pérennisoside XV, du pérennisoside XVIII & XIX, du désacyl-pérennisoside I, II & III, du désacyl-pérennisoside VIII & IX et du désacyl-pérennisoside X et XI.

12. Composition à base de plantes selon la revendication 11, comprenant en outre éventuellement un ou plusieurs des composés de saponine bellisisosides C & E, bellisisoside D, pérennisosides I & II, pérennisoside VII, pérennisosides VIII & IX, pérennisosides X & XI, pérennisoside XVI, pérennisaponine A et pérennisaponines G ; H.

13. Composition à base de plantes selon l'une quelconque des revendications 1 à 12, ayant une valeur de pH comprise entre environ 4,0 et 6,5.

14. Composition à base de plantes selon l'une quelconque des revendications 1 à 13, comprenant en outre un conservateur choisi parmi le déshydroacétate de sodium, l'acide 2-hydroxybenzoïque, le 1-phényl-1-propanol, le Dermosoft^{®} 700B, l'alcool phénéthylique et le benzoate de sodium.

15. Formulation pharmaceutique ou cosmétique, comprenant une composition à base de plantes selon l'une quelconque des revendications 1 à 14.

16. Formulation cosmétique selon la revendication 15.

17. Formulation pharmaceutique comprenant une composition à base de plantes selon l'une quelconque des revendications 1 à 14 pour une utilisation dans la prévention ou le traitement thérapeutique de cernes périorbitaires et/ou d'un œdème périorbitaire, laquelle formulation pharmaceutique agit comme un stimulateur d'autophagie, un activateur d'hème oxygénase, un déclencheur de production de VEGF-C et/ou un réducteur de synthèse de mélamine.

18. Formulation pharmaceutique pour une utilisation selon la revendication 17, dans laquelle les cernes sous les yeux et/ou les gonflements sont dus à l'un quelconque parmi l'hérédité, des allergies, un eczéma (dermatite atopique), une dermatite de contact, un rhume des foins/une rhinite allergique, un stress, une carence en fer ou d'autres troubles du fer, un traumatisme mineur, des pleurs, une rétention de fluide, une exposition excessive au soleil, un frottement et un grattage des yeux, et un médicament.

19. Utilisation non thérapeutique d'une composition à base de plantes selon l'une quelconque des revendications 1 à 14 en tant que stimulateur d'autophagie, activateur d'hème oxygénase, déclencheur de production de VEGF-C et/ou réducteur de synthèse de mélamine, pour la prévention ou le traitement cosmétique de cernes périorbitaires et/ou d'yeux bouffis.

20. Utilisation selon la revendication 19, dans laquelle les cernes sous les yeux et/ou les gonflements sont dus à l'un quelconque parmi un amincissement de la peau et/ou une perte de graisse et de collagène avec l'âge (vieillissement), les choix de style de vie, un manque de sommeil/une fatigue, un sommeil excessif, une rétention de fluide, et l'influence du rythme circadien selon le style de vie et les conditions de vie.

21. Formulation pharmaceutique selon l'une quelconque des revendications 15 à 18, comprenant une composition à base de plantes selon l'une quelconque des revendications 1 à 12 à une concentration de 3 à 5 % (en poids/poids) du poids total de la formulation totale.

22. Formulation pharmaceutique ou cosmétique selon l'une quelconque des revendications 15 à 21, destinée à être appliquée par voie topique.

23. Formulation pharmaceutique ou cosmétique selon la revendication 22, laquelle formulation est une crème, une pommade, une émulsion (lait), un tonique (lotion), un bâtonnet, une dispersion, une formulation comprenant un nettoyant tensioactif, une solution, de l'eau micellaire, un gel, un masque, un tampon en tissu humide ou un masque en tissu humide.

24. Utilisation selon l'une quelconque des revendications 19 à 20, pour une application par voie topique.

25. Utilisation selon la revendication 24 pour une application par voie topique sous la forme d'une crème, d'une pommade, d'une émulsion (lait), d'un tonique (lotion), d'un bâtonnet, d'une dispersion, d'une formulation comprenant un nettoyant tensioactif, d'une solution, d'eau micellaire, d'un gel, d'un masque, d'un tampon en tissu humide ou d'un masque en tissu humide.
